# EUROPEAN PATENT SPECIFICATION

(11) **EP 4 154 798 B1**
(45) Date of publication and mention of the grant of the patent: **09.07.2025**
(21) Application number: 21198717.7
(22) Date of filing: 24.09.2021
(51) Int. Cl.: A61B 5/00, A61B 5/055

(54) **APPARATUS FOR DETERMINING A POSTURE OF A PATIENT FOR AN EXAMINATION TO BE PERFORMED WITH A MEDICAL EXAMINATION APPARATUS AND CORRESPONDING METHOD**
VORRICHTUNG ZUR BESTIMMUNG DER KÖRPERHALTUNG EINES PATIENTEN ZUR UNTERSUCHUNG MIT EINEM MEDIZINISCHEN UNTERSUCHUNGSGERÄT UND ENTSPRECHENDES VERFAHREN
APPAREIL PERMETTANT DE DÉTERMINER LA POSTURE D'UN PATIENT EN VUE D'UN EXAMEN AVEC UN APPAREIL D'EXAMEN MEDICAL ET PROCÉDÉ CORRESPONDANT

(43) Date of publication of application: 29.03.2023
(73) Proprietor: Siemens Healthineers AG, 91301 Forchheim (DE)
(72) Inventor: Kapoor, Ankur, Plainsboro, 08536 (US); Mailhe, Boris, Plainsboro, 08536 (US); Nadar, Mariappan S., Plainsboro, 08536 (US); Singh, Vivek, Princeton, 08540 (US); Tamersoy, Birgi, 91052 Erlangen (DE)
(74) Representative: Siemens Healthineers Patent Attorneys

(56) References cited:
- CN-A- 112 741 643
- CN-U- 206 924 167
- KR-A- 20210 091 902
- US-A1- 2009 300 843
- US-A1- 2018 140 270

## Description

The invention concerns a positioning apparatus according to claim 1, with further embodiments being defined by dependent claims 2 -13.

Further, the invention concerns a computer-implemented method according to claim 14 and a computer program product according to claim 15.

Putting the patient into a correct body pose is a fundamental requirement both in diagnostic and interventional imaging. In most cases, the anatomy of interest will be most visible/accessible when the patient is positioned in a specific pose and imaged from a specific viewpoint.

To date, patient positioning still is a very manual process mainly done by the healthcare personnel supervising or conducting the examination. As a first step of the process, the healthcare personnel will think about an optimal posture for the patient or the relevant body parts of the patient. Finding a suitable posture is a complicated task that requires a lot of experience to get it right at once. One reason for this is that there typically is a huge variety of different examinations that may be performed with one and the same medical examination modality. Taking a medical imaging modality such as an MRI device as an example, imaging the lung of a patient may require a totally different posture than, e.g., the knee. Another reason is that individual patients may have very different requirements regarding placement on the patient table. For example, it may be the case that rather large patients have to be positioned in a different manner than patients of a normal size. The patient's body circumference may also require a specially adapted arrangement on the patient table. Another issue lies in the fact that the healthcare personnel also has to consider the overall adjustment range of the patient table. Moreover, once the patient is placed on the patient table, the posture of the patient has to be fixed using a variety of support equipment (e.g., foams, pillows, etc.). Once the patient pose is manually checked and confirmed, then further stabilizing equipment (e.g., sandbags) are used for ensuring that the patient pose is not altered during the scan.

In summary, therefore, there is a wide variety of basic circumstances that have to be taken into account when placing a patient correctly on a patient table, but which are often difficult for the healthcare personnel to assess from a causal point of view. As a result, positioning of the patient on the patient table and adjusting the patient table is frequently based on "trial and error". The patient is experimentally positioned on the patient table and then the patient table is moved to the examination position is approached on a test basis. This is a very time-consuming process. As a result, such a procedure increases the occupancy times of the devices. Moreover, it may cause agitation in patients who may already be tense in view of the examination they will have to go through. Moreover, this may result in "re-takes" if the positioning was not suitable.

In interventional setups, correct patient positioning becomes even more important since in most cases pre-operational data is aligned with the intra-operational data and this can be done most accurately if the patient pose matches between the two. For example, in radiotherapy, both finding the optimum pose during the planning stage, and then keeping the patient pose constant between the planning stage and the therapy stage is central for a successful treatment.

CN 206924167 U discloses an adaptable patient table with a control device which enables to automatically position a patient.

US 2018 / 0 140 270 A1 discloses methods and systems for automatically adjusting a position of a table configured to be positioned in a bore of a medical imaging device. The table may be adjusted according to one or more of a table elevation, an anatomical reference, and a start scan location. The table elevation, anatomical reference, and start scan location may be obtained from a selected imaging protocol.

Accordingly, it is an object of the present invention to provide apparatus, systems and methods facilitating imaging and/or interventional and/or therapeutic examination procedures to be performed on a patient. In particular, it is an object to provide apparatus, systems and methods enabling an improved conduction of imaging and/or interventional and/or therapeutic examinations on a patient with improved precision and reduced expenditure of work for the involved healthcare personnel. In other words, it is an object to provide apparatus, systems and methods improving the treatment success for imaging and/or interventional and/or therapeutic examinations on a patient. In particular, it is an object of the present invention to provide apparatus, systems and methods enabling an easier positioning of a patient for an imaging and/or interventional and/or therapeutic examination to be performed on that patient.

This object is solved by apparatus for adjusting a posture of a patient for an imaging and/or interventional and/or therapeutic examination of the patient, corresponding examination systems, and corresponding methods according to one or more of the independent claims. Alternative and/or preferred embodiments are subject of the dependent claims.

In the following, the technical solution according to the present invention is described with respect to the claimed systems as well as with respect to the claimed methods. Features, advantages, or alternative embodiments described herein can likewise be assigned to other claimed objects and vice versa. In other words, claims addressing the inventive method can be improved by features described or claimed with respect to the systems and vice versa. In these cases, functional features of the method may be embodied by objective units, modules, or elements of the system and vice versa. In the following, the terms "in particular", "preferably", and "optional" are used to indicate optional features. Likewise, explanations and/or expressions given in brackets relate to optional features. Also features introduced with the phrase "in other words" generally relate to optional features or modifications. According to an aspect, a positioning apparatus for adjusting a posture of a patient for an imaging and/or interventional and/or therapeutic examination of the patient is provided. The apparatus comprises a patient support device, an interface unit, and a computing unit. The patient support device is adjustable such that one or more body segments of the patient may be arranged and/or fixed in a predetermined posture. The interface unit is configured to provide examination information indicative of an examination to be performed at the patient. The computing unit is configured to receive the examination information via the interface unit. The computing unit is further configured to determine, based on the examination information, a posture of one or more body segments of the patient for the examination), wherein the examination information includes an information about a previous posture previously determined for the patient in a prior examination, wherein the information about the previous posture comprises medical image data depicting a region of interest targeted in the prior examination, and the computing unit is configured to determine the posture by identifying a region of interest of the patient targeted in the prior examination from the information about the previous posture by processing the medical image data acquired in the prior examination and determining the posture based on the identified region of interest. The computing unit is configured to generate one or more control signals, the one or more control signals being suited to control the patient support device such that the patient support device is adjusted such that the one or more body segments of the patient are arranged and/or fixed in the determined posture. The computing unit is further configured to provide the control signals to the patient support device, preferably via the interface unit.

The imaging and/or interventional and/or therapeutic examination of the patient may be carried out by a medical examination apparatus. Another expression for examination may be procedure. The medical examination apparatus and/or the examination may require arranging the patient or at least certain body segments of the patient in a certain posture to be successful. A body segment may generally relate to a part or section of the patient's body. For instance, a body segment may be a body part of the patient, such as the head, the arms, the legs, the chest region, the thorax region, and so forth. According to further examples, a body segment may relate to sub-sections or organs of these body parts such as an upper arm, the pelvis, the heart, the lung lobes, a coronary artery, and so forth.

An imaging examination may comprise imaging one or more body segments of the patient with a medical imaging modality such as a magnetic resonance device. An interventional examination may comprise conducting an interventional procedure on one or more body segments of the patient with an interventional modality such as a surgical robot. A therapeutic examination may comprise conducting a therapeutic treatment of one or more body segments of the patient using a therapeutic modality such as a radiotherapy device.

The patient support device may generally be configured to receive and support a patient during an examination. The patient support device may comprise an adjustable and/or deformable patient table. The patient support device may comprise an adjustable and/or deformable receiving surface for receiving one or more body segments of the patient, body parts of the patient, or the patient as whole. In particular, adjustable and/or deformable may mean that a 3D contour or topography of the receiving surface may be adjusted - so that the posture of a patient or a body segment of the patient may be indirectly influenced or set by adjusting the patient support device (if the patient is placed on the patient support device). According to some examples, the patient support device may be internally adjustable in the sense that individual parts of the patient support device may be moved relative to one another to adjust a posture of a patient or a body part of the patient supported by the patient support device. According to some examples, the patient support device may be internally adjustable in the sense that the topography of the receiving surface is adapted. In other words, adjusting the patient support device may comprise adapting an arrangement state of the aforementioned parts. In particular, the patient support device may be configured to be automatically adjustable, i.e., adjustable without requiring manual interactions of a user or the like. To this end, the patient support device may comprise an automated and controllable adjustment mechanism configured to adjust the patient support device such that one or more body segments of the patient may be arranged and/or fixed in a predetermined posture. The automated adjustment mechanism may be configured to receive control signals directed to adjust the patient support device and implement the control signals by accordingly activating individual actuators and/or driving units comprised in the automated adjustment mechanism.

The interface unit may generally be configured to facilitate, in particular bi-directional, communication between the components of the positioning apparatus, such as the computing unit and the patient support device, and external components such as a medical examination apparatus or any data bases for gathering information relevant for conducting the examination and setting the posture of the patient. The interface unit may comprise a hardware and/or software interface, for example a PCI bus, a USB interface, a Fire-Wire interface, a ZigBee or a Bluetooth interface. The interface unit may comprise an interface of a communications network, wherein the communications network may include a Local Area Network (LAN), for example an Intranet, or a Wide Area Network (WAN) such as the Internet. Accordingly, the interface unit may comprise a LAN interface or a Wireless LAN interface (WLAN or Wi-Fi). Furthermore, the interface unit may also comprise a plurality of said interfaces in combination.

The computing unit can be embodied as a centralized or decentralized controller of the positioning apparatus. According to some examples, the computing unit may also be integrated in a controller of medical examination apparatus. According to other examples, the computing unit may also resume functions directed to controlling the medical examination apparatus. The computing unit can include one or more control devices and/or one or more processors. The computing unit can be implemented as a local or cloud-based computing server. The computing unit can advantageously include at least one user interface. The user interface can include an input element, for example a keyboard and/or a joystick and/or a capacitive and/or resistive input panel, which enables the positioning apparatus to be controlled by a user through a corresponding operator input and/or to input information relevant for finding a suitable posture of one or more body segments of the patient. The user interface can further include a display element, for example a monitor or a display. The display element can be embodied for the graphical depiction of one or more results of the determination regarding the positional state of the patient for a user.

The examination information may generally comprise data characterizing the examination to be performed on the patient. This may include an information what type of examination is to be performed and what kind, type or make of medical examination apparatus will be used. Further, the examination information may include an indication of the region of interest or target for the examination. The region of interest may be a body region of the patient that is to be subjected to the examination by the medical examination apparatus. The region of interest can comprise one or more body segments of the patient. Herein, the region of interest can depend both on the planned examination per se and the geometry of the patient. For example, the region of interest can be defined as the thorax region of the patient if, for example, the chest cavity of a patient is to be imaged. As a further example, the region of interest can indicate, that, in particular, a rear side of the lung is to be imaged. According to further examples, the region of interest may relate to an organ on which an interventional procedure is to be performed. Furthermore, the region of interest may relate to a body region that is to be irradiated in a radiotherapy session. The region of interest can describe a two-dimensional subarea or a three-dimensional subspace of the patient. If, for example, a whole-body scan is to be performed, the region of interest can also correspond to the entire patient. Thus, the type of examination to be performed has an influence on the region of interest - and thus on the body segments of the patient that are relevant and how these should be positioned. Thus, the region of interest in turn determines which positional states of the patient are suitable for the examination to be performed and which are less suitable.

According to some implementations, the region of interest may be generic for the type of examination. According to other implementations, the regions of interest may be specific for the patient and already be defined in the coordinate system of the patient and/or the medical examination apparatus.

The posture of one or more body segments may, in particular, relate to the positional state of the body segments relative to the patient positioning apparatus and/or the patient support device and/or the medical examination apparatus and/or the rest of the patient's body. The positional state can describe how (anatomical) landmarks are, or should be, arranged relative to the patient positioning apparatus and/or the patient support device and/or the medical examination apparatus and/or the rest of the patient's body. Such landmarks may, in particular, be the contour (silhouette, outline) of the patient or body segments of the patient, i.e., for example, head, eyes, thorax, chest, legs, joints etc. The posture or positional state can further relate to an orientation of the patient or the patient's body segments. The orientation can, in particular, comprise the relative orientation of one or more body segments of the patient with respect to patient positioning apparatus and/or the patient support device and/or the medical examination apparatus and/or the rest of the patient's body or the general arrangement of the patient, i.e., for example, supine position, lateral position right, lateral position left, prone position etc.

According to some examples, upon determining the posture of the one or more body segments, the computing unit may further be configured to identify the one or more body segments (which are relevant for the respective examination) based on the examination information prior to determining the posture thereof.

According to some implementations, the computing unit may further be configured to determine a region of interest for the examination to be performed (that is a body region of the patient to be targeted in the examination) based on the examination information. The computing unit may further be configured to identify the one or more body segments and/or determine the posture of the one or more body segments based on the region of interest (the identified body region).

According to some examples, the computing unit may be further configured to establish a registration between the medical examination apparatus and the positioning apparatus (in particular: the patient support device) and/or the patient and the positioning apparatus when determining the posture of the one or more body segments of the patient. In other words, a coordinate system of the medical examination apparatus can advantageously be registered with the coordinate system of the positioning apparatus (in particular: the patient support device) and/or a coordinate system of the patient can advantageously be registered with the coordinate system of the positioning apparatus (in particular: the patient support device). Hereby, a corresponding spatial position in the coordinate system of the positioning apparatus (in particular: the patient support device) can be assigned to each position in the region of interest. The registrations enable that posture and location details can be easily converted into each other, and this can facilitate, for example, the output of suitable control signals for actuation of the patient support device. For instance, a region of interest as provided for in the examination information and given in the coordinate system of the patient can easily be mapped onto the coordinate system of the positioning apparatus facilitating the generation of appropriate control signals.

The control signals are generally suited to control the patient support device such that it resumes a predetermined spatial arrangement that brings the patient received on the patient support device into the determined posture (in the sense that the one or more body segments of the patient are arranged in the determined posture). The control signals may be high-level control signals just indicating a desired spatial arrangement which will be further processed by the patient support device to appropriately control individual actuators. According to some examples, the control signals may already comprise distinct instructions for the individual actuators of the patient support device, however. Control signals may, for instance, be generated based on predetermined characteristic curves which translate a given posture into control parameters for appropriately deforming the patient support device.

The above features synergistically work together to autonomously position a patient for an examination using an automatically adjustable patient support device. By taking examination information into account, a posture of one or more body segments of the patient is determined that is specifically adapted to the examination to be performed. With that, suitable positional states of the patient can be determined more quickly, and unfavorable positional states can be avoided or at least corrected more efficiently. This not only improves the success rate of the examination but also reduces the workload of the healthcare personnel. Moreover, especially less experienced healthcare personnel are provided with assistance in finding the correct posture for the patient. What is more, since the autonomous adjustment as proposed here can be performed faster with improved robustness and repeatability, also the degree of utilization of the medical examination facility may be improved.

According to some examples, the interface unit is configured to be in data communication with a data base, in which data base the examination information is stored. Further, the interface unit is configured to retrieve the examination information from the data base and/or provide the examination information to the computing unit.

Further, providing the examination information from the data base may involve querying the data base for the examination information. For instance, the computing unit may be configured to generate a data query for retrieving the examination information from the data base. According to some examples, the data base may be part of the positioning apparatus or the medical examination system, for instance, as part of an examination planning module or scheduling module for occupancy planning of medical examination apparatus. Specifically, such scheduling module may be configured to host an electronic work list of the examinations to be performed. Upon selecting one of these examinations, the examination information may be automatically loaded from the data base. According to some examples, the data base may likewise be part of an external healthcare information system. The healthcare information system may, for example, be embodied as a hospital information system or radiology information system and may, in particular, contain a scheduling module as described before.

In that case, the positioning apparatus and/or the medical examination system may also be connected to the healthcare information system.

The retrieval of the examination information enables to automatically collect relevant information to identify the relevant body segments for the examination and determine an appropriate posture on that basis without requiring user input. In particular, such appropriate posture can be seen as a coarse determination which may be further refined. This improves the user-friendliness of the system. Alternatively, the examination information (or parts thereof) may also be prespecified by a user input, however.

According to some examples, the interface unit is further configured to provide patient information, the patient information comprising one or more properties or characteristics of the patient (which preferably are of potential relevance for the examination). The computing unit is further configured to receive the patient information via the interface unit and determine the posture of one or more body segments of the patient for the examination additionally based on the patient information.

According to some implementations, the patient information may include information regarding one or more geometric properties of the patient. Herein, the geometric properties can generally relate to dimensions of the patient. For example, the geometric properties can include the (body) height, the (body) circumference, the position of one or more anatomical landmarks of the patient, dimensions of parts or segments of the patient, such as, for example, the torso length or the circumference of the abdomen or chest. Herein, the position of the one or more anatomical landmarks can include a plurality of anatomical landmarks relative to one another - for example the hip-to-head distance. The position of the one or more landmarks can further comprise the absolute position of the landmarks in relation to the patient and/or the regions of interest.

Taking account patient information enables the determination concerning the posture to be better adapted to the geometry of the patient. For example, the body height and/or body circumference of the patient can be taken into account during the determination, whereby an evaluation or calculation of a positional state can be individually adapted to the physiological conditions of the patient.

According to some examples, the computing unit may be configured to identify the region of interest and/or the relevant body segments additionally based on the patient information.

This is based on the realization that the region of interest depends not only on the examination to be performed, but also on the individual characteristics of the patient. If, for example, a thorax scan is to be performed, the region of interest (the relevant body segments) will be embodied differently depending on the body height, torso length and/or torso circumference. This is taken into account by dynamically determining the region of interest (the relevant body segments) in dependence on the patient information. Thus, the one or more body segments can be better estimated when determining a suitable positional state.According to some examples, the patient information may include information regarding one or more constraints relevant to the examination of the patient. Constraints relevant to the examination may relate to information beyond the aforementioned geometric properties, which cannot be determined, or can only be determined with difficulty, by measuring the patient, but which could possibly influence the positioning of the patient on the positioning apparatus. In particular, such constraints can relate to psychosomatic restrictions of the patient, such as a restricted mobility of one or more body parts, claustrophobic impairments, possible states of confusion, the degree of anesthesia or the responsiveness of a patient. Further constraints relevant to the examination can result from the need for ventilation/dialysis or the administration of contrast medium during the examination.

Taking account of constraints relevant to the examination enables the determination concerning the posture to be even better adapted to the circumstances of the patient and the examination to be performed. For example, this enables account to be taken of the fact that, if possible, a claustrophobic patient should not be pushed head-first into the opening of a gantry of an imaging device or that the need for ventilation requires an adapted posture. Moreover, patients with decreased levels of responsiveness may require to be more tightly fixed in the respective posture, e.g., by additionally adjusting the patient support device such that not only the posture can naturally be assumed by the patient but also the movement of the patient is restricted.

According to some examples, the interface unit is configured to be in data communication with a data base, in which data base the patient information is stored. Further, the interface unit is configured to retrieve the patient information from the data base and/or provide the patient information to the computing unit.

Further, providing the patient information from the data base may involve querying the data base for the patient information. For instance, the computing unit may be configured to generate a data query for retrieving the patient information from the data base.

The data base storing the patient information may or may not be the same data base that stores the examination information. According to some examples, the data base may be part of the positioning apparatus or the medical examination system, for instance, as part of an examination planning module or scheduling module for occupancy planning of medical examination apparatus. According to some examples, the data base may likewise be part of an external healthcare information system. The healthcare information system may, for example, be embodied as a hospital information system or radiology information system and may, in particular, contain a scheduling module as described before.

The retrieval of the patient information enables to automatically collect relevant information to identify the relevant body segments for the examination and determine an appropriate posture on that basis without requiring user input. This improves the user-friendliness of the system. Alternatively, the patient information (or parts thereof) may also be prespecified by a user input, however.

According to some examples, the positioning apparatus may further comprise a detection unit configured to provide detection results by measuring the patient prior and/or during the examination and to provide the detection results to the interface unit. The interface unit is configured to provide the detection result to the computing unit. The computing unit is configured to receive the detection result and to extract at least part of the patient information from the detection result.

The detection unit can be configured to scan at least part of the surface of the patient. In particular, the detection unit can be configured to scan at least part of the surface of the patient in a contactless manner. The detection unit can include at least one electromagnetic sensor configured for the aforementioned tasks. The detection unit and/or the computing unit may, in particular, be configured to calculate a three-dimensional contour of the scanned surface. The detection unit and/or the computing unit may, in particular, be embodied to identify one or more anatomical landmarks based on the scanned surface, in particular, based on the calculated contour.

Herein, the measurement of the patient may, in particular, take place by means of optical measuring methods. According to some implementations, for this purpose, the sensor facility can include an optical measuring facility. The optical measuring facility can, for example, be embodied in the form of a camera facility and/or a triangulation system and/or a laser-scanning facility. Herein, the camera facility may, in particular, include a 3D-camera facility such as a stereo camera system configured to detect a three-dimensional image of the patient. The laser-scanning facility can also generate a three-dimensional model of the patient by time-of-flight measurement of emitted and re-detected laser beams. The laser-scanning facility may, in particular, be embodied as a LIDAR system. The optical measuring facility can further include a time-of-flight measuring system ("time-of-flight camera"). In some implementations, the optical measuring facility may be embodied to scan a surface by means of structured lighting. One advantage of the aforementioned optical measuring systems lies in a comparatively quick and equally accurate detection of basic geometric properties. The optical measurement of the patient may be followed by further processing steps. Such processing steps can, for example, include image data evaluation methods for the image data recorded with the camera facility, for example, in order to extract the three-dimensional contour of the patient, dimensions or the position of one or more anatomical landmarks of the patient and provide these as part of the patient information.

A conceivable alternative to optical measurement of the patient is measurement based on contact with the patient. In some implementations, the detection unit can, for example, include a pressure-sensitive sensor facility. This pressure-sensitive sensor facility can, for example, be embodied as a pressure-sensitive mat which is arranged on the patient support device and configured to determine the patient information (in particular one or more geometric properties of the patient) from the pressure load exerted by the patient.

According to further implementations, the detection unit may include a combination of one or more of the aforementioned components, which creates redundancies and ensures reliable detection of the patient information. For example, within the detection unit, a camera facility and/or laser-scanning facility can be combined with a pressure-sensitive sensor facility. In other words, the detection unit may include at least one of a camera facility, a laser-scanning facility, and/or a pressure-sensitive sensor facility.

According to some examples, the positioning apparatus may comprise a detection unit configured to provide a detection result indicative of a current posture of one or more body segments of the patient arranged on the patient support device and to provide the detection result to the interface unit. The interface unit is configured to provide the detection result to the computing unit. The computing unit is configured to receive the detection result, to extract an information about the current posture of one or more body segments of the patient from the detection result, and to determine the one or more control signals additionally based on the information about the current posture. According to some implementations, the computing unit may further be configured to compare the information about the current posture with the determined posture and to generate the one or more control signals based on the comparison.

The detection unit may be configured essentially as described before. In particular, the detection unit may comprise a camera facility, a laser-scanning facility, and/or a pressure-sensitive sensor facility. Moreover, the detection unit according to these examples may comprise built-in sensor circuitry comprised in the patient support device which sensor circuitry is configured to provide information about the arrangement state of the patient support device.

The ascertainment of the current posture enables this to be taken into account when generating the control signals.

Further, this enables to detect if the patient has moved out of position and, if so, determine a correction in terms of adapted control signals. According to some implementations, the current posture is defined relative to the patient support device and/or in the coordinate system of the patient support device. The information about the current posture can describe how (anatomical) landmarks are arranged relative to the patient support device.

According to some examples, the examination information includes an information about a previous posture previously determined for the patient in a prior examination and the computing unit is configured to determine the posture based on the previous posture. Additionally, or alternatively, the information about a previous posture may also be comprised in the patient information. According to some implementations, the computing unit is configured to determine the posture such that the determined posture matches the previous posture, and preferably such that the determined posture corresponds to or is even identical to the previous posture. According to some implementations, the prior examination relates to an imaging examination of the patient and the examination relates to an interventional and/or therapeutical examination of the patient based on the imaging examination. Specifically, according to some implementations, the imaging examination may relate to a planning stage of the interventional and/or therapeutical examination of the patient. According to other implementations, the prior examination may relate to an imaging examination and the examination may also relate to an imaging examination, wherein the same or at least a similar imaging region (region of interest) of the patient is to be imaged. In other words, the examination may relate to a follow-up imaging examination with respect to a prior imaging examination. According to other implementations, the prior examination may relate to a interventional and/or therapeutical examination and the examination may relate to an imaging examination to assess the success of the interventional and/or therapeutical examination. In other words, the examination may relate to a follow-up imaging examination with respect to a prior interventional and/or therapeutical examination.

Generally speaking, the previous examination may define one or more targets, i.e., regions of interest, for the examination. In particular, the prior examination and the examination may comprise or target the same or at least a similar region of interest.

The information about the previous posture may be provided by one or more of: a text string specifying a body region of the patient targeted in the prior examination, control signals for controlling the patient support device generated for the prior examination, medical image data depicting a body region (region of interest) targeted in the prior examination, a machine-readable indication about the posture as determined in the prior examination (e.g., by the computing unit), a machine-readable indication or log about the settings of the patient support device as used in the prior examination and/or the like. In particular, such information about the previous posture may be (or may have been) automatically provided (outputted) by the detection unit and appended to the patient data.

According to some examples, the computing unit may be configured to identify a body region (or region of interest) of the patient targeted in the prior examination from the information about the previous posture and determine the posture based on the identified body region (or region of interest).

Thereby, according to some implementations, the computing unit may be configured to identify the body region (or region of interest) by processing medical image data acquired in the prior examination (if the information about the previous posture comprises such data). Specifically, the computing unit may be configured to recognize organs and/or anatomical landmarks in medical image data and to identify the body region (or region of interest) based on the recognized organs and/or anatomical landmarks.

Taking into account a previous posture used in a prior examination enables to robustly resume the previous posture in a follow-up examination. In radiotherapy, for instance, both finding the optimum pose during the planning stage, and then keeping the patient pose constant between the planning stage and the therapy stage is central for a successful treatment.

According to some examples, the computing unit is further configured to save an indication about the determined posture in the patient and/or examination information of the patient (e.g., by adding this information to the patient and/or examination information stored in the healthcare information system). Thereby, the indication about the determined posture may have the same form as the indication about the previous posture discussed before. In particular, this may include storing the determined posture and/or the control signals in the patient and/or examination information.

Storing an indication about the determined posture in the patient and/or examination information makes it possible to readily reuse this information when a follow-up examination of the patient is to be performed in the future.

According to some examples, the examination is conducted with a medical examination apparatus, wherein the medical examination apparatus comprises a predetermined operation range in which the examination is to be performed. Further, the computing unit is configured to determine the posture additionally based on the operation range.

Taking account of the operation range enables an improved estimation to be made as to which postures are suitable for the examination to be performed and which are not suitable. Herein, the operation range may, in particular, be taken into account in conjunction with the adjustment range of the patient support device (see below) which provides a complete picture of the maximum relative movement possibilities within the examination system.

According to some examples, the patient support device comprises a predetermined adjustment range and the computing unit is further configured to determine the posture additionally based on the adjustment range.

Taking account of the adjustment range enables a better estimation to be made as to whether a posture can be at all reached and is therefore suitable for the examination to be performed. For example, it may be the case that the adjustment range of individual parts of the patient support device is not sufficient to move the body segments far enough.

According to some examples, the computing unit is further configured to ascertain if the determined posture of the one or more body segments has been reached and, if so, provide a signal indicative that the determined posture of the one or more body segments has been reached. According to some implementations, the signal may be provided to the medical examination apparatus via the interface unit (so that the medical examination apparatus may start with the examination). According to other implementations, the signal may be provided to a user of the positioning apparatus via an appropriate user interface. According to some implementations, the computing unit may be further configured to ascertain if the determined posture of the one or more body segments has been reached based on a comparison of the determined posture with the information about the current posture as described elsewhere herein.

With that, the examination may either automatically be started or the correct positioning of the patient may at least be communicated to the user further increasing the user-friendliness of the apparatus.

According to some examples, the computing unit is configured to host at least one machine-learned function configured to determine a posture of one or more body segments of the patient based on input data. Input data may be examination information and/or patient information as herein described. Further, the computing unit is configured to determine the posture by applying the at least one machine-learned function to at least the examination information (and, optionally, the patient information).

According to some examples, the computing unit is configured to host a plurality of different machine-learned functions each configured and/or trained to determine a posture of one or more body segments of the patient based on input data. Input data may be examination information and/or patient information as herein described. Further, the computing unit is configured to select one of the hosted machine-learned functions depending on at least the examination information (and, optionally, the patient information). Further, the computing unit is configured to determine the posture by applying the selected machine-learned function to at least the examination information (and, optionally, the patient information).

According to some implementations, the computing unit may be configured to select the one machine-learned function based on the type of examination to be performed and the medical examination apparatus to be used. Further, the computing unit may be configured to select the one machine-learned function additionally based on the patient information and, in particular, one or more patient characteristics extracted from the patient information, such as the gender, weight, height class, age of the patient and so forth.

The machine-learned functions may be algorithms trained for different purposes. Preferably they have been trained with training data of different examinations and/or groups of patients, and/or with training data relating to different medical examination apparatus. The functions may also be trained with different types of data (i.e., examination information and/or patient information), e.g., one function may be trained with examination information indicating a region of interest by way of medical image data, while another function may be trained to extract corresponding information from a medical report comprising text.

Each machine-learned function may be trained to identify regions of interest or body segments of the patient which are to be optimally positioned and to determine a posture for these body segments. There may be a number of specialized machine-learned functions for different types of examinations, different medical examination apparatus, different patient cohorts, different patient support devices and so forth. Preferably, there are in total more than 10 different machine-learned functions used, especially more than 30 or even more than 50.

The machine-learned functions may be present in the computing unit itself or in a memory used by the computing unit. For example, information about the architecture and parameters of the machine-learned functions are present in a memory and a chosen machine-learned function is downloaded from the memory into (a random access memory of) the computing unit.

The selecting step is conceived for automatically selecting one of the available machine-learned functions for the respective use case. Preferably, the machine-learned function is selected that fits best to the available data and examination scheme and is therefore supposed to provide the best results. The selection may be based on a predefined selection scheme, on information available to be processed by the machine-learned functions, and/or on the respective patient or use case. The selection scheme may be a table stored in a memory of the computing unit or a decision tree hardwired in the computer program product running in the computing unit. Alternatively, the selection scheme may be implemented by a dedicated machine learned model itself.

A machine-learned function, in general, may be seen as mapping input data to output data thereby fulfilling a certain learned task - here the determination of a posture of one or more body segments of a patient for an examination to be performed on the patient. Machine-learned functions may be based on one or more neural networks (e.g., deep neural networks, recurrent neural networks, convolutional neural networks, convolutional deep neural networks, adversarial networks, deep adversarial networks and/or a generative adversarial networks etc.) and/or other algorithms including Bayesian networks, decision trees, random forest schemes, support vector machines, linear or logistic regression models, gradient averaging, k-means clustering, Q-learning, genetic algorithms and/or association rules or other suitable models and any combination of the aforesaid. Instead of the term "neural network" the term "neuronal net" can also be used. The output generated by the machine-learned functions may depend on one or more parameters of the machine-learned functions. In general, a machine-learned function may comprise a manifold of parameters. For instance, the parameters may relate to parameters deeply embedded in the machine-learned function such as the weights in artificial neural networks. Further, the parameters may relate to "superordinate" parameters (often denoted as "hyperparameters") which govern the overall behavior and training of the prediction model. Hyperparameters can be real-valued (e.g., learning rate, width of decision boundaries), integer-valued (e.g., number of layers), binary (e.g., whether to use early stopping or not), or categorical (e.g., choice of optimizer). The one or more parameters of the machine-learned function can be determined and/or be adjusted by training. The term "machine-learned function" as used throughout the application relates to models or algorithms which already underwent training, i.e., the parameters of which have already been determined and/or adjusted by training.

The different types of machine-learned functions which can be hosted in the computing unit may relate to structurally and/or functionally and/or topologically different types or kinds or variants of machine-learned functions. As such, different types may rely on different structures or architectures. For instance, neural networks may comprise a layer structure, while random forest schemes comprise a decision tree structure. Further, the machine-learned functions may fulfill different functions. While some may be configured for feature learning by processing image data, others may be configured to classify data or provide numerical predictions. Further, the machine-learned functions may differ with regard to the learning processes. For instance, one type of machine-learned functions may infer parameters from using labeled data pairs by ways of supervised learning. Examples include various kinds of neural networks, decision trees, or Bayesian networks. Other types of prediction models may support unsupervised learning where previously unknown patterns are found in data sets without pre-existing labels or semi-supervised learning. Examples include deep believe nets, hierarchical clustering, or k-means. In terms of machine learning paradigms, yet a third type of machine-learned function relates to models supporting reinforcement learning which is concerned with how models ought to take actions in an environment so as to maximize some notion of cumulative reward. Examples include Q-learning or learning classifier systems. Furthermore, the machine-learned function may differ with respect to different metrics optimized during the training process. For instance, for decision tress and tree-based methods in general, these metrics may include information gain, Gini impurity, gain ratio or accuracy.

The determination and/or the adjustment of the one or more parameters of a machine-learned function during training may occur during the initial creation or compilation of the machine-learned function from scratch. It may further occur during later training of an already trained machine-learned function, for instance during re-training or incremental machine learning or transfer learning. Other terms for machine-learned function may be machine-learned model, trained mapping specification, mapping specification with trained parameters, function with trained parameters, algorithm based on artificial intelligence, machine learned algorithm. Training, in general, may be based on a pair made up of training input data and associated training output data. A trainable machine-learned function is applied to the training input data for creating output data. In particular, the determination and/or the adjustment can be based on a comparison of the output data and the training output data. After training (performance evaluation and deployment), the machine-learned function may process new unseen data to generate output data according to what it has been trained for. Thus, the training may be seen as implicitly defining the field of application and/or the task the machine-learned function has to fulfill.

Specifically, the training input data and the training output data may be gained from a past imaging and/or interventional and/or therapeutic examinations of patients. The training input data may relate to the examination information and/or patient information available for the patient at the point in time of the examination. The training output data may relate to a posture of the patient that proved useful and that enabled the successful completion of the examination. The posture may for instance be acquired essentially as herein described in connection with the "information about the current posture", i.e., using a detection unit. In addition, training may comprise further training during deployment of the machine-learned function. For instance, it is conceivable that a user makes adaptations to the posture determined by the machine-learned function in order to further improve the posture (e.g., for making the posture more comfortable for the patient). These user adaptations may be fed back to the machine-learned function to further adapt/optimize its parameters by ways of further training and/or incremental machine learning. According to other examples, the training input and training output data may be gained from simulations and/or computations. In particular, output data may comprise a simulated optimal or at least suitable posture of a patient for certain training input data. The training input data may comprise real or virtual examination information and/or patient information.

The usage of machine-learned functions in general has the advantage that a more comprehensive and faster screening of the available information can be made. In this regard, machine-learned functions may even identify patterns in the available data that are not accessible for a human. The optional selection of the best machine-learned function for the respective case has the additional advantage of further improved results.

According to some examples, upon selecting the machine-learned function, the input data (i.e., examination information and/or patient information) may be searched for predefined, examinations, medical examination modalities, data types and/or for values of predefined data types. For example, it may be determined whether an imaging examination shall be made. Using this information, an appropriate machine-learned function may then be selected as a next step.

According to some examples, the patient support device comprises a plurality of support segments configured to support different body segments of the patient. The support segments are adjustable relative to one another such that one or more body segments of the patient can be arranged and/or fixed at a predetermined posture. The one or more control signals are suited to adjust at least a part of the support segments such that the one or more body segments of the patient supported by the support segments are arranged and/or fixed at the determined posture.

A plurality of support segments configured to support different body segments of the patient may mean that each support segment is configured to support different body segments of the patient with respect to the respective other support segments. This may mean that the individual support segments are arranged at different positions of the patient support device and/or have different shapes. According to some implementations, the support segments (together) may define a receiving surface of the patient support device for receiving the patient. In other words, each support segment may comprise an individual receiving surface for receiving one or more body segments of the patient or a part of a body segment of the patient or a part of one or more body segments of the patient. The individual support surfaces may be conceived as together defining the receiving surface of the patient support device. Since the support segments are adjustable relative to one another, this leads to a receiving surface the 3D-contour or topography of which is adjustable or deformable. Adjustable may mean that the spatial arrangement of the support segments can be adapted within the patient support device (i.e., in the reference system of the patient support device). In particular, this may mean that the spatial arrangement of the individual receiving surfaces of the support segments may be adjusted with respect to the patient support device. Adjusting a support segment may comprise setting a height of the individual receiving surface of the support segment, an inclination of the individual receiving surface of the support segment, an orientation of the individual receiving surface of the support segment, and/or a lateral position of the individual receiving surface of the support segment, and/or the like.

The usage of suchlike support segments has the advantage that the receiving surface of the patient support device can be shaped with high precision according to the examination and the patient.

According to some examples, the support segments (all or a part of the support segments) are configured such that they may be adjusted independently from one another. According to other implementations, at least a part of the support segments are linked to one another such that an adjustment of one support segment will affect the adjustment of those support segments linked thereto. While the former may have the advantage of more degrees of freedom for defining the receiving surface of the patient support device, the latter may be beneficial in that jumps between individual receiving surfaces are smoothed making the receiving surface more comfortable for the patient.

According to some examples, at least a part of the support segments are pillar-type support segments respectively comprising a height adjustable pillar, column, or piston. The pillar-type support segments may be placed at different positions within the patient support device. The pillar-type support segments may be supported against a base plate of the patient support device. The tips of the pillar-type support segments may be configured as individual support surfaces, respectively, or may be configured to respectively hold the individual support surface of the respective support segment. The usage of pillar-type support segments may have the advantage that the receiving surface may be adjusted with great precision. At the same time, the pillar-type support segments provide huge degrees of flexibility as to the design of the receiving surface.

According to some examples, the patient support device comprises a plurality of the pillar-type support segments arranged in an array within the patient support device. The array may span part of the receiving surface of the patient support device or the entire receiving surface of the patient support device. The array may comprise 10 to 400 pillar-type support segments and preferably 20 to 200 or 50 to 100 pillar-type support segments. The pillar-type support segments may be regularly arranged with the array. Preferably, the array may comprise a plurality of rows of pillar-type support segments, each row comprising a plurality of pillar-type support segments. According to some implementations there may be 10 to 20 rows, each row comprising 5 to 15 pillar-type support segments.

The usage of an array of pillar-type support segments may have the advantage of uniformly distributed adjustment options. Using the array nearly any suitable shape of the receiving surface may be adopted. In particular, such pillars may also be used to effectively restrict the free moving space of a body segment of the patient and thereby fix the one or more body segments in the determined posture. For instance, this may be achieved by letting one or more pillar-type support segments protrude with respect to other pillar-type support segments such that a body part of the patient abuts against the protruding pillar-type support segments.

According to some examples, at least some of the support segments are provided with actuators which can be activated by the control signals in order to adjust the associated support segments.

According to some implementations, all of the support segments may be provided with an individual actuator. The actuators may comprise a drive means for adjusting the spatial arrangement and/or deformation of the associated support segment. The actuators may comprise electric, hydraulic, and/or pneumatic drive means. The actuators may comprise sensing circuitry configured to detect the control value or current state of the respective actuator which may, e.g., enable the computing unit to deduce the current spatial arrangement or deformation state of the patient support device.

The usage of suchlike actuators enables a tight control of the deformation state of the receiving surface of the patient support device. At the same type, new deformation states may be rapidly reached.

According to another aspect a medical examination system is provided. The medical examination system comprises a positioning apparatus according to the aspect, examples, and implementations as herein descried. Further, the medical examination system comprises a medical examination apparatus for conducting the imaging and/or interventional and/or therapeutic examination.

The medical examination apparatus may comprise a medical imaging modality generally configured to image an anatomical region (one or more body segments, a region of interest) of the patient. According to some implementations, the medical imaging modality may comprise a computed tomography (CT) device, an X-ray device, a C-arm X-ray device, a magnetic resonance tomography (MRT) or magnetic resonance imaging (MRI) device, a positron-emission tomography (PET) device, a single-photon emission computed tomography (SPECT) device, an ultrasound (US) device, and/or combinations thereof. Further, the medical examination apparatus may comprise an interventional and/or therapeutic modality. The interventional and/or therapeutic modality may generally be configured to carry out an interventional and/or therapeutic medical procedure at the patient (i.e., on one or more body segments of the patient or a region of interest of the patient). The interventional and/or therapeutic modality may comprise an interventional device to perform an interventional procedure on one or more body segments of the patient. According to some implementations, the interventional modality may be embodied by a biopsy device, e.g., comprising a biopsy needle, a minimal invasive device, e.g., comprising one or more minimal invasive actuators for examining and/or treating one or more body segments of the patient, or the like. According to some examples, the interventional modality may comprise a robotic device, such as a catheter robot, a minimal invasive surgical robot, an endoscopy robot, or the like. Further, the interventional and/or therapeutic modality may comprise a radiotherapy or radiation therapy device for irradiating, in particular ionizing, radiation onto one or more body segments or regions of interest of the patient.

According to another aspect, a computer-implemented method to determine a posture of a patient for an imaging and/or interventional and/or therapeutic procedure to be performed at the patient is provided. The patient is arranged at a patient support device during the procedure and the patient support device is adjustable/controllable such that one or more body segments of the patient may be arranged and/or fixed at a predetermined posture. The method comprises a plurality of steps. One step is directed to provide an examination information indicative of an examination to be performed at the patient. A further step is directed to determine a posture of one or more body segments of the patient based on the examination information, wherein the examination information includes an information about a previous posture previously determined for the patient in a prior examination, wherein the information about the previous posture comprises medical image data depicting a region of interest targeted in the prior examination, and the computing unit is configured to determine the posture by identifying a region of interest of the patient targeted in the prior examination from the information about the previous posture by processing the medical image data acquired in the prior examination and determining the posture based on the identified region of interest. A further step is directed to generate one or more control signals, the one or more control signal being suited to control the patient support device such that the patient support device is adjusted such that the one or more body segments of the patient are arranged and/or fixed in the determined posture. A further step is directed to provide the control signals to the patient support device.

The method may be further modified according to the aspects, examples and implementation herein described with respect to the apparatus and systems. In particular, the configurations of the apparatus and systems may be mapped onto corresponding method steps. Likewise, the advantages described in connection with the apparatuses and systems may also be realized by the corresponding method steps. Specifically, the method may further comprise a step directed to provide patient information, the patient information comprising one or more properties of the patient relevant for the examination, wherein in the step of determining the posture, the posture of one or more body segments of the patient is determined additionally based on the patient information.

According to another aspect, the present invention is directed to a computer program product comprising program elements which induce a computing unit of a positioning apparatus and/or a medical examination apparatus to perform the steps according to the methods as herein described, when the program elements are loaded into memories of the respective computing units.

The realization of the invention by a computer program product has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances. Characteristics, features and advantages of the above described invention, as well as the manner they are achieved, become clearer and more understandable in the light of the following description and embodiments, which will be described in detail with respect to the figures. This following description does not limit the invention on the contained embodiments. Same components, parts or steps can be labeled with the same reference signs in different figures. In general, the figures are not drawn to scale. In the following:
FIG. 1 schematically depicts an embodiment of a system for performing an imaging and/or interventional and/or therapeutic examination;
FIG. 2 schematically depicts a patient support device according to an embodiment;
FIG. 3 schematically depicts a top view of a patient support device according to a further embodiment;
FIG. 4 schematically depicts a side view of the patient support device according to the further embodiment;
FIG. 5 depicts a flowchart illustrating a method for determining and adjusting a posture of a patient using an adjustable patient support device according to an embodiment;
FIG. 6 depicts a flowchart illustrating a method for determining and adjusting a posture of a patient using an adjustable patient support device according to a further embodiment; and
FIG. 7 depicts a flowchart illustrating a method for determining and adjusting a posture of a patient using an adjustable patient support device according to a further embodiment.

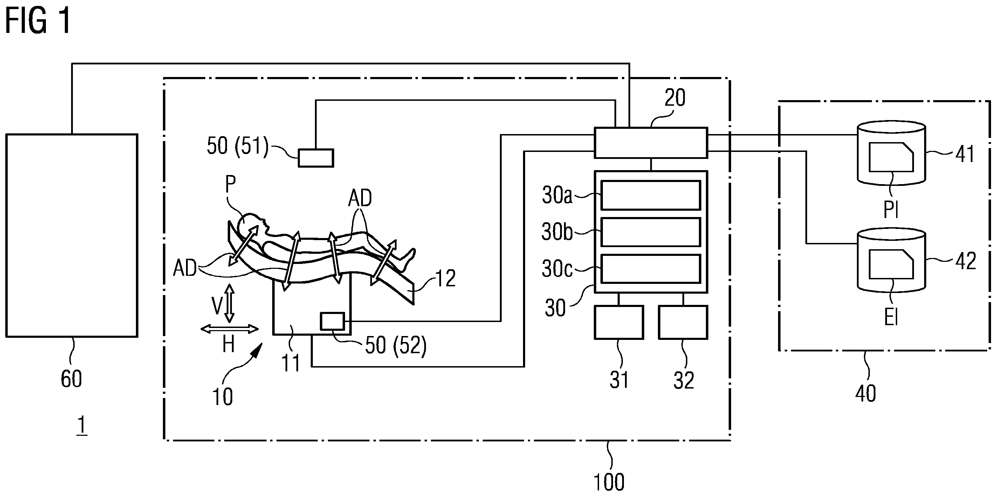

**Figure 1** depicts a system 1 for performing an imaging and/or interventional and/or therapeutical examination on a patient P. System 1 comprises a medical examination apparatus 60 and a positioning apparatus 100 for positioning patient P or at least relevant body segments of patient P for an imaging and/or interventional and/or therapeutical examination or procedure that shall performed on patient P using medical examination apparatus 60.

Medical examination apparatus 60 may comprise a medical imaging modality 61. Medical imaging modality 61 may generally be configured to image an anatomical region of patient **P.** Imaging the anatomical region of patient P with medical imaging modality 61 may be suited to identify one or more targets (regions of interest) within that body region for an interventional and/or therapeutical treatment of patient P. In particular, such targets may be pathological changes within the imaged anatomical region. For instance, such pathological changes may relate to lesions within the anatomical regions, such as cancerous growths, masses, stenoses, and so forth. According to some implementations, medical imaging modality 61 may comprise a computed tomography (CT) device, an X-ray device, a C-arm X-ray device, a magnetic resonance tomography (MRT) device, a positron-emission tomography (PET) device, a single-photon emission computed tomography (SPECT) device, an ultrasound (US) device, and/or combinations thereof.

Further, medical examination apparatus 60 may comprise an interventional and/or therapeutic modality 62. Interventional and/or therapeutic modality 62 may generally be configured to carry out an interventional and/or therapeutic medical procedure at a patient P. In particular, such interventional and/or therapeutic procedure may be directed to one or more targets (regions of interest) identified with medical imaging modality 61. Interventional and/or therapeutic modality 62 may comprise an interventional device to perform an interventional procedure on a target anatomical region of patient P. According to some implementations, the interventional device may be embodied by a biopsy device, e.g., comprising a biopsy needle, a minimal invasive device, e.g., comprising one or more minimal invasive actuators for examining and/or treating target anatomical regions of patient P, or the like. According to some examples, the interventional device may comprise a robotic device, such as a catheter robot, a minimal invasive surgical robot, an endoscopy robot, or the like. Further, interventional and/or therapeutic modality 62 may comprise a radiotherapy or radiation therapy device for irradiating, in particular ionizing, radiation onto a target anatomical region of patient P. According to such implementations, interventional and/or therapeutic modality 62 may comprise one or more medical linear accelerators or other radiation sources.

It is noted that the number and arrangement of devices and/or components of medical examination apparatus 60 shown in Fig-ure 1 and explained above are provided as an example. In practice, medical examination apparatus 60 may include additional devices and/or components, fewer devices and/or components, different devices and/or components, or differently arranged devices and/or components than those shown in Figure 1. Specifically, medical examination apparatus 60 may comprise either medical imaging modality 61 or interventional and/or therapeutic modality 62. Moreover, medical imaging modality 61 and/or interventional and/or therapeutic modality 62 may each comprise a plurality of different devices. For instance, medical imaging modality 61 may comprise a plurality of, preferably different, imaging devices.

In some implementations, medical examination apparatus 60 may have a predefined operation range in which the imaging and/or interventional and/or therapeutic examination is to be performed. Taking medical imaging modality 61 as an example, the operation range may be defined by the imaging region of the medical imaging device(s) comprised in medical imaging modality 61. For instance, the imaging region and thus the operation range may be limited to the bore of an MRT device. As far as interventional and/or therapeutic modalities 62 are concerned, the operation range may be determined by the reach of an interventional device such as a medical robot or the irradiation region of a radiotherapy device.

Positioning apparatus 100 may comprise a patient support device 10 and a computing unit 30. Further, positioning apparatus 100 may comprise an interface unit 20 for interfacing computing unit 30 with patient support device 10 and other components of positioning apparatus 100 and/or external components (such as medical examination apparatus 60).

Patient support device 10 is generally configured to receive, support, and/or position patient P or dedicated body segments of patient P during the imaging and/or interventional and/or therapeutic examination patient P is supposed to go through. According to some implementations, patient support device 10 may comprise a patient couch or patient table 12 configured to receive patient P or at least body segments of patient P. Patient table 12 may be connected to a base 11 such that base 11 supports patient table 12 with patient P. Patient table 12 and/or base 11 may be configured such that patient support device 10 can move patient P in a horizontal direction H and in a vertical direction V perpendicular to H. In addition to that, patient table 12 and/or base 11 are automatically adjustable such that one or more body segments of patient P may be arranged and/or fixed in a predetermined posture. In other words, patient table 12 and/or base 11 are configured such that they can be automatically brought into a predetermined spatial arrangement, which spatial arrangement is suited to arrange and/or fix one or more body segments of patient P in a predetermined posture. In other words, patient support device 10 is controllable to adjusts itself to bring patient P or body segments of patient P into a desired posture. In particular, this may involve bringing patient table 11 into a desired spatial arrangement or shape so that the suchlike adjusted or deformed patient table (i.e., the 3D contour or profile of the patient-receiving surface(s) of patient table 12) supports patient P in the predetermined posture.

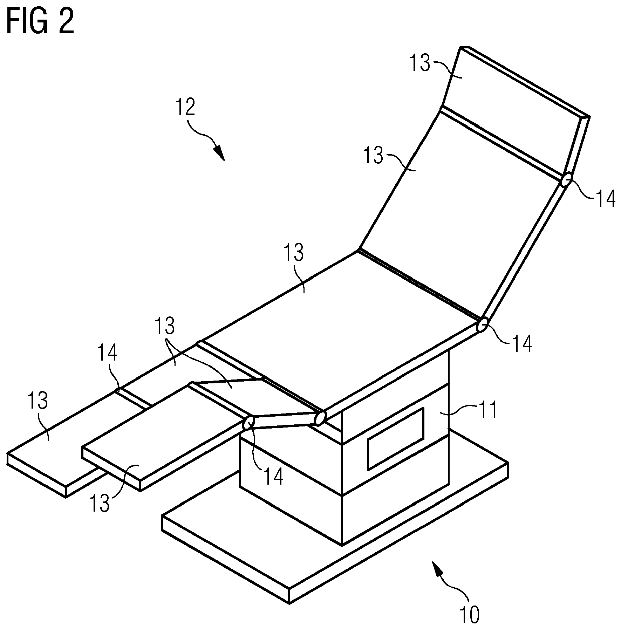

To this end, patient table 12 and/or base 11 may have a plurality of internal degrees of freedom which may allow to move body segments of patient P relative to one another, in particular, along a plurality of internal adjustment directions AD. According to some implementations, this may be realized by providing patient table 12 with a plurality of support segments 13, wherein each of support segments 13 is configured to support different body segments of patient P. By making these support segments 13 adjustable relative to one another, e.g., by providing at least some of support segments 13 with controllable actuators 14, one or more body segments of patient P can be arranged and/or fixed at a predetermined posture. Further details as to possible implementations and modes of operation of patient support device 10 will be provided in connection with Figures 2 to 4 below.

Self-speaking, the adjustability of patient support device 10 is not unlimited. Rather, all components have limits in their travel paths which may depend on the current relative spatial arrangement of the components of patient support device 10. These limits determine the overall adjustable range of patient support device 10.

According to some implementations, patient support device 10 is controlled by computing unit 30. Specifically, computing unit 30 may be configured to process relevant information in order to determine a suitable posture for patient P for the examination to be performed. The thus determined posture may then be translated into appropriate control signals for patient support device 10, i.e., control signals which may prompt patient support device 10 to assume the spatial arrangement suited to bring patient P in the determined posture. Relevant information on the basis of which computing unit 30 may determine a suitable posture may comprise any one of patient information PI, examination information EI, the adjustment range(s) of patient support device 10, the operation range of medical examination apparatus 60, information about the current posture of the patient CPI, and/or any combination of the aforesaid. In this regard, patient information PI may contain patient parameters relevant to the examination (for example body height, body circumference, torso length, gender etc.) and/or patient-constraints (for example psychosomatic restrictions such as claustrophobic anxiety, restrictions of the patient's locomotor system, etc.). Examination information EI may comprise an information about the examination to be performed such as the body segment(s) of interest for the examination, the duration of the examination, requirements as to how steady the patient's posture has to be, and/or the like.

To gather suchlike relevant information, computing unit 30 may be configured to interface, via interface unit 20, with further components of positioning apparatus 100 or external components which could provide this information. For instance, examination information EI and/or patient information PI may be retrieved from a medical information system 40 comprising one or more data bases 41, 42. Travel and operation ranges may directly be obtained from patient support device 10 and medical examination apparatus 60, respectively. Further, computing unit 30 may be in data exchange with various detection and sensor facilities 51, 52, which may be configured to measure patient information PI or a current posture of patient P on patient support device 10, e.g., by scanning patient P.

Computing unit 30 can be embodied as a central control unit, for example, as a control device with one or more processors. Alternatively, computing unit 30 can be embodied as part of medical examination apparatus 60. According to further implementations, the functionalities of computing unit 30 described below can also be distributed in a decentralized manner between a plurality of processing units or control devices.

Computing unit 30 may comprise a data retrieval module 30a configured to retrieve examination information EI, patient information PI, the adjustment range(s) of patient support device 10, the operation range of medical examination apparatus 60, and/or information about the current posture of the patient. Further, computing unit 30 may comprise an analysis module 30b configured to determine a posture for at least one or more body segments of patient P for an examination to be performed with medical examination system 60 based on the information retrieved by retrieval module 30a. In addition, computing unit 30 may comprise a control module 30c. Control module 30c may be configured to control patient support device 10. Specifically, control module 30c may be configured to translate the posture determined by analysis module 30b into appropriate control signals which are suited to automatically bring patient support device 10 into a spatial arrangement which is suited for arranging and/or fixing body segments of patient P in the determined posture if patient P is placed on patient support device 10. Of note, the designation of the distinct modules 30a-30c is to be construed by way of example and not as limitation. Specifically, modules 30a-30c may be virtual modules. Moreover, modules 30a-30c may be integrated to form one single real or virtual unit or can be embodied by computer code segments configured to execute the corresponding method steps running on computing unit 30.

Computing unit 30 may further include a user interface 31, 32. The user interface 31, 32 can include an input unit 31 (e.g., a keyboard) and/or a display unit 32, (e.g., a monitor and/or display). Herein, the input unit 31 can be integrated in the display unit 32, for example in the form of a capacitive and/or resistive input display. The input unit 32 can be embodied to receive inputs from a user, in particular user input concerning patient information PI and/or examination information EI. According to some implementations, user inputs may be received via a graphical user interface.

Furthermore, in some implementations, patient positioning apparatus 100 may comprise a detection unit 50. Detection unit 50 may comprise a plurality of detection devices or sensors 51, 52. Detection unit 50 may be configured to provide detection results from which patient information PI or at least parts of the patient information PI of a patient P undergoing an examination may be derived, such as the body height or the body circumference of patient P to be examined. Alternatively, or additionally, detection unit 50 may be configured to provide detection results form which an information about a current posture of patient P accommodated on patient support device 10 may be derived.

Detection unit 50 may comprise a detection device 51 embodied for contactless scanning of at least part of the surface of patient P. For this purpose, detection device 51 can be embodied to detect electromagnetic radiation, in particular, to detect electromagnetic radiation in a low-frequency spectral range compared to X-rays, for example in the visible or infrared spectral range. According to some implementations, detection device 51 can comprise one or more photographic cameras or one or more video cameras. Specifically, detection device 51 may comprise a 3D camera embodied, for example, as a stereo camera or as a time-of-flight camera. According to some examples, detection device 51 can be configured to scan a surface by way of structured lighting. In that case, detection device 51 may additionally include a lighting unit for generating structured lighting on at least part of the surface of patient P and detection device 51 may be configured to detect the radiation reflected from the surface of patient P.

According to some examples, such detection device 51 may be attached to or integrated in medical examination apparatus 60 (e.g., in the gantry of a CT device). Detection device 51 can furthermore be positioned somewhere else in the room in which medical examination apparatus 60 is located. For example, it can be attached to or suspended from the ceiling or placed on a stand such as a tripod. According to some examples, medical examination apparatus 60 can be positioned centrally above patient P or centrally above patient support device 10.

According to some examples, detection device 51 can also be configured to ascertain patient information PI by "pre-scanning" patient P before patient P is arranged on patient support device 10, for example, when patient P enters the examination room.

According to some examples, detection unit 50 may comprise a sensor device 52 configured as a touch- or pressure sensitive sensor facility. For instance, sensor device 52 may be embodied as an array of a plurality of pressure-sensitive elements arranged on patient support device 10, the position of which can advantageously be registered with patient support device 10. In addition to patient information PI such as the body length and body circumference of patient P, this enables to infer the relative positional state of patient P with respect to patient support device 10. According to some implementations, sensor device 52 may be embodied by a pressure sensitive mat arranged on patient support device 10, the pressure sensitive mat comprising the aforementioned array of pressure-sensitive elements. According to other implementations, the pressure-sensitive elements may be included in the adaption mechanism of patient support device 10. For instance, each or at least a part of the aforementioned support segments 13 and/or actuators 14 may comprise a pressure-sensitive element configured and arranged such that the load of the respective body segment of patient P on the respective support segment 13 can be determined form the respective sensor signal.

As mentioned, positioning apparatus 100 may be interfaced with healthcare information system 40 in order to receive examination information EI and/or patient information PI therefrom. Healthcare information system 40 may be a local system within the premises of a healthcare organization. Moreover, healthcare information system 40 may be cloud based. Healthcare information system 40 may comprise one or more standardized components such as a picture archiving and communication system (PACS), a radiology information system (RIS), a laboratory information system (LIS), a hospital information system (HIS), or an electronic medical record system (EMR). For communication with healthcare information system 40, interface unit 20 may be configured according to an appropriate standard compatible with the respective healthcare information system, such as the HL7 FHIR standard. According to some implementations, healthcare information system 40 may comprise a scheduling system for occupancy planning of medical examination apparatus 60 or include a corresponding planning module for occupancy planning. Alternatively, such scheduling system may be part of medical examination system 1.

Healthcare information system 40 may include one or more data bases 41, 42. Data bases 41 and 42 may be respectively configured to store examination information EI and/or patient information PI, respectively. Data bases 41 and 42 may be realized as cloud storages. Alternatively, data bases 41 and 42 may be realized as local or spread storages. According to some implementations, there may be separate data bases 41, 42 for examination information EI and patient information PI, respectively. For instance, patient information PI as introduced above may be stored in the electronic medical record of patient P. Accordingly, data base 42 storing patient information PI may be an EMR-data base or part of an EMR-system. In contrast to that, examination information EI may be stored in a data base 41 which is part of an aforementioned scheduling system or hospital information system. According to other embodiments, examination information EI and patient information PI may be stored in one data base. According to further implementations, one or more of data bases 41, 42 may also be part of medical examination system 1.

Data connections between system components (for example the medical examination apparatus 60, patient positioning apparatus 100, computing unit 30 detection and sensor facilities 51, 52 and/or healthcare information system 40) can take place via one or more suitable data interfaces, with interface unit 20 embodying the data interface of computing unit 30. Data interfaces such as interface unit 20 may include a hardware and/or software interface, for example a PCI bus, a USB interface, a FireWire interface, a ZigBee interface or a Bluetooth interface. Further, data interfaces such as interface unit 20 can include an interface of a communication network, wherein the communication network can include a local area network (LAN), for example an intranet or a wide area network (WAN). Accordingly, the one or more data interfaces can include a LAN interface or a wireless LAN interface (WLAN or WiFi).

**Figure 2** schematically depicts a possible realization of patient support device 10 according to an embodiment. Patient support device 10 comprises a plurality of adjustable support segments 13 being part of patient table 12. Support segments 13 may be adjusted in that their position in space and/or relative to one another may be changed. In other words, this configuration of patient table 12 with a plurality of support segments 13 makes patient table 12 deformable. According to the example shown in Figure 2, support segments 13 are configured such that individual support segments 13 correspond to individual body parts or body segments of patient P, such as upper and lower legs, head, thorax, or chest. The position and arrangement of individual support segments 13 may be adjusted by drive units or actuators 14 which may, for instance be integrated into the joints between segments 13. Actuators 14 may be individually controlled such that patient table 12 resumes an appropriate arrangement for receiving and/or fixing patient P in a posture determined by control unit 30.

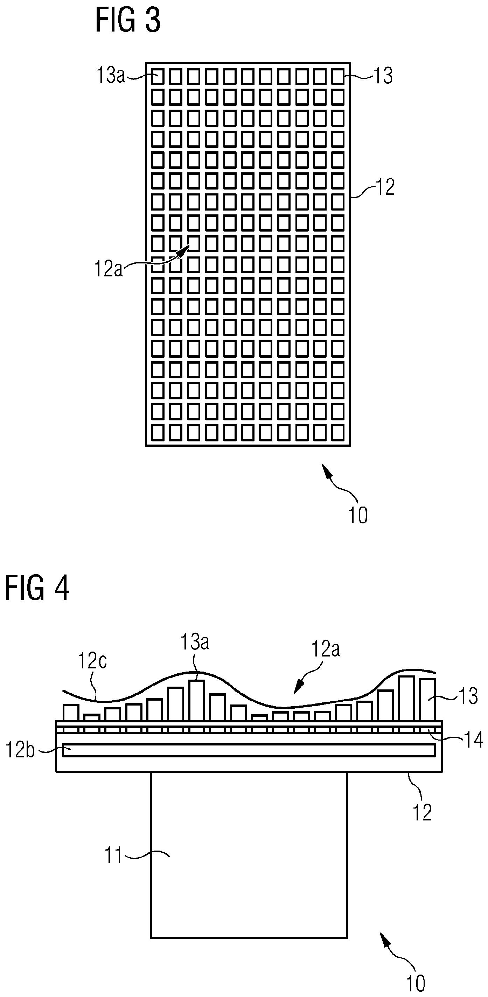

**Figures 3 and 4** depict a possible realization of patient support device 10 according to a further embodiment, wherein Figure 3 shows a top view of patient support device 10 and Figure 4 shows a side view. Patient table 12 according to this embodiment comprises a plurality of support segments 13 which are arranged in a two-dimensional array (c.f., Figure 3). Support segments 13 may be seen as small pistons or pillars the height of which can be individually adjusted by dedicated actuators 14. The top sides 13a of segments 13 together define a receiving surface 12a for receiving patient P. Preferably, top sides 13a have a surface which is much smaller than corresponding body parts of patient P. Accordingly, patient table 12 may comprise a plurality of rows of support segments 13, with each row comprising a plurality of support segments 13. By consequence, receiving surface 12a may be sensibly adjusted to optimally fit to patient P and the determined posture. In other words, patient table 12 according to this embodiment allows for a multi-contour adaptation or deformation of patient table 12. According to some examples the array of support segments 13 may have 10 to 20 rows and each row may comprise 4 to 15 individual support segments 13.

For each support segment 13 there may be a dedicated actuator 14 configured to at least adjust the height of the corresponding segment 13. Actuators 14 may be embodied by linear motors, (hydraulic or pneumatic) piston setups, servomotors and the like. Like in the previous embodiment, actuators 14 may be individually controllable in order to bring support segments 13 into the appropriate height as, e.g., determined by control unit 30.

Support segments 13 and actuators 14 may be supported by a base plate 12b of patient table 12. In other words, base plate 12b is configured to receive the array of support segments 13. Base plate 12b may have dimensions essentially corresponding to typical body dimensions of a patient P and being compatible with spatial constraints of medical examination apparatus 60. For instance, base plate 12b may be 2 meters in length and 1 meter in width. Top sides 13a of segments 13 are typically considerably smaller than base plate 12b. According to some implementations, top sides 13a may have a width of 5 to 25 centimeters and/or a length of 5 to 25 centimeters and/or a diameter of 5 to 25 centimeters. The contour of top sides 13a may be round, rectangular, or quadratic. Thereby all support segments 13, and accordingly top sides 13a, may have the same shape and/or dimensions. According to other implementations, differently shaped and/or dimensioned support segments 13 and top sides 13a may be used throughout patient table 12. To increase the comfort for patient P, top sides 13a may be rounded and, e.g., have an essentially hemispheric shape. Furthermore, patient table 12 may comprise a mat 12c covering and thereby cushioning the array of support segments 13 towards patient P.

The embodiments for patient support device 10 shown in Fig-ures 2, 3 and 4 are meant as examples and should not be construed to limit the disclosure. There are various additional ways to realize patient support devices 10 with deformable patient tables 12 being adjustable such that one or more body segments of patient P may be arranged and/or fixed in a predetermined posture. For instance, patient table 12 may comprise one or more bladders which may be filled with air or other suitable fluids, e.g., using an appropriate compressor. In the systematic of the present application such a bladder would be an adjustable support segment and the compressor would be the corresponding actuator. By selectively inflating or deflating one or more bladders of patient table 12, patient table 12 may likewise be brought into a shape which is suited to support patient P in an appropriate posture. According to other examples, patient table 12 may comprise one or more flexible chambers filled with a material having a solid, dimensionally stable state and a moldable state, in particular, at least partially liquid and/or gaseous state, which states are linked by reversible phase transitions which can be steered, e.g., by energy input. The chambers would again correspond to the above support segments while the energy source for inducing the phase transitions would be a corresponding actuator.

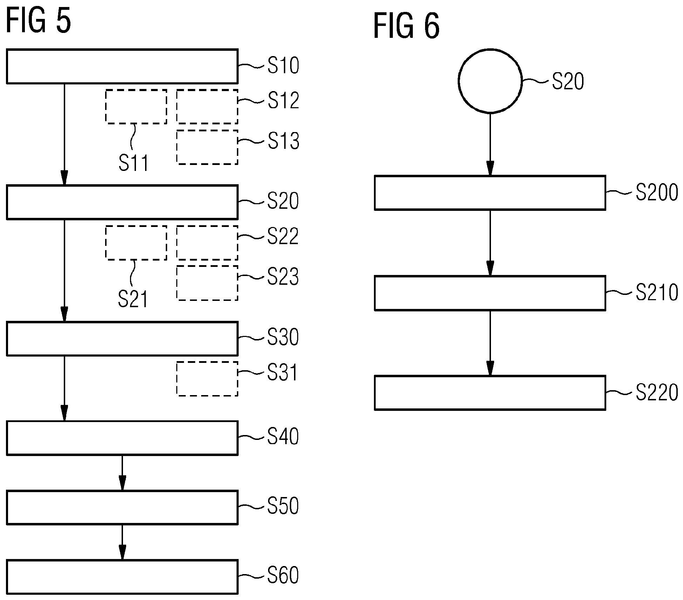

**Figure 5** depicts a method for determining a posture of patient P for an imaging and/or interventional and/or therapeutic procedure patient P is to be subjected to according to an embodiment. The order of the steps does not necessarily correspond to the numbering of the steps but may also vary between different embodiments of the present invention. Further, individual steps or sequences of steps may be repeated. At step S10, examination information EI of patient P is received at computing unit 30 via interface unit 20. Step S10 may comprise optional step S11 of providing examination information EI. Examination information EI may be provided by a user of medical examination system 1 by inputting examination information EI into input unit 31. Further, examination information EI may be provided by healthcare information system 40. According to further examples, examination information EI may be provided by a scheduling system for occupancy planning of examination system 1 (which may be part of medical examination system 1 or healthcare information system). According to some examples, step S10 may further comprise optional sub step S12 directed to actively requesting or querying examination information EI from the relevant sources (i.e., the user, pertinent data bases 42 of healthcare information system 40, or a scheduling system). According to some implementations, step S12 may be initiated by manually selecting patient P in a worklist by a user of medical examination system 1. According to some examples, step S10 may further comprise optional sub step S13 of processing the information received at step S10 in order to extract relevant examination information EI therefrom. Specifically, an electronic medical record of patient P may be requested using a suitable electronic identifier (for example via the patient's name or the patient's ID) and automatically searched for examination information EI.

At step S20, patient information PI of patient P is received at computing unit 30 via interface unit 20. Like with step S10, step S20 may comprise an optional sub step S21 of providing patient information PI. In addition to the sources for examination information EI, patient information PI may additionally be provided by detection unit 50. Specifically, patient information PI may be acquired by means of detection device 51 by scanning patient P. Scanning patient P can take place before or after patient P has been arranged on patient support device 10. Similar to step S10, patient information PI may be actively request by computing unit 30 in an optional sub step S22. Further, step S20 may comprise optional sub step S23 directed to processing the information received at step S20 in order to extract relevant patient information PI therefrom.

It is to be noted that a suited posture of body segments of patient P may also already be calculated solely based on examination information EI, i.e., without taking patient information PI into account. According to such implementations, step S20 may be omitted. Vice versa, it is also conceivable to determine postures of body segments of patient P solely based on patient information PI without considering examination information EI. This may be the case if it is a priori clear which type of examination is to be performed. According to such implementations, step S10 may be omitted.

At step S30, a posture of one or more body segments of patient P is determined based on the available information regarding patient P and the examination to be performed. Relevant information in this respect may be patient information PI, examination information EI, the operation range of medical examination apparatus 60, and/or the adjustable range of patient support device 10. The goal of step S30 is to determine a reasonably good posture of one or more body segments, body parts or the entire patient for the examination to be performed on patient P. The body segments which are to be accordingly positioned are, in particular, those body segments of patient P which are affected by the examination. In order to determine a posture of body segments of patient P at step S30, computing unit 30 may rely on one or more deterministic algorithms configured to take in patient information PI, examination information EI, the operation range of medical examination apparatus 60, and/or adjustable range of patient support device 10 and determine said posture on that basis.

In particular, such algorithms may be rule-based. According to other examples, computing unit 30 may also be configured to host one or more machine learned functions configured to output said posture based on patient information PI, examination information EI, the operation range of medical examination apparatus 60, and/or the adjustable range of patient support device 10. According to some implementations, computing unit 30 may also rely on a Kalman Filter for that purpose.

Specifically, it is conceivable that computing unit 30 hosts a plurality of different machine learned functions each configured to determine a posture of body segments of a patient P based on the above input data but for different types of examinations. In other words, there may be different machine-learned functions for different examinations. While one machine-learned function has been optimized to predict suitable postures for interventional procedures using a surgical robot, another machine-learned function may have been trained to determine postures for imaging procedures using an MR device and so forth. In that case, step S30 may comprise an optional step S31 of selecting one of the machine-learned functions based on examination information EI in order to determine the posture with the selected machine-learned function.

In step S40, one or more control signal(s) are generated for patient support device 10, which are capable of deforming/adjusting patient support device 10 such that patient P will be brought into the posture determined at step S30. In particular, the one or more control signal(s) can be capable of controlling individual actuators 14 so as to move individual support segments 13 into the right positions in space in accordance with the determined posture. For generating appropriate control signals, computing unit 30 may be provided with information about the current spatial conformation or adjustment state of patient support device 10, i.e., the current positions of support segments 13. The current positions of support segments 13 may serve as starting values from which any further movement of support segments 13 may be planned to derive appropriate control signals. In other words, the control signals may be based on a current adjustment state of patient support device 10. The current adjustment state may be measured using appropriate sensing devices (not shown) which may, for instance, be integrated in actuators 14.

At step S50, the one or more control signal(s) are provided to patient support device 10. At step S60, segments 13 are moved based on the one or more control signals so that patient support device 10 is brought into the correct position that lets patient P assume the posture as determined at step S30. Step S60 may be performed before patient P has been placed on patient support device 10 or while patient P lies on patient support device 10. Moving patient support device 10 according to the control signals may involve a feedback of the current adjustment state of patient support device 10 to computing unit 30. With that, the current adjustment state may be constantly checked and used in step S40 to adapt the generation of the control signals if needed.

**Figure 6** depicts optional method steps for receiving patient information PI by using detection unit 50. The steps of Fig-ure 6 may optionally be part of step S20. Detection unit 50 may comprise detection device 51 and/or sensor device 52. As explained above, detection device 51 and sensor device 52 may rely on different measurement principles. While detection device 51 can be conceived as a scanning system scanning patient P from a distance, sensor device 52 may be conceived as a contact-based measurement system relying on pressure sensitive elements, for instance. The basic workflow of Figure 6 is the same for both measurement principles, however. At step S200, detection results are acquired from detection device 51 and/or sensor device 52. As the case may be, this can be scanning data acquired by scanning patient P or load data indicative where and how patient P puts weight on patient support device 10. At step S210, the detection results are received at computing unit 30 via interface unit 20. At step S220, the detection results are processed to derive patient information PI therefrom. Taking detection device 51 as an example, step S220 may involve calculating a three-dimensional contour of patient P based on the scanning result and determine, based on the three-dimensional contour, one or more body dimensions of patient P. If the detection result relates to load data, step S220 may comprise extracting, e.g., a height of patient P based on where patient P exerts a load on patient support device 10.

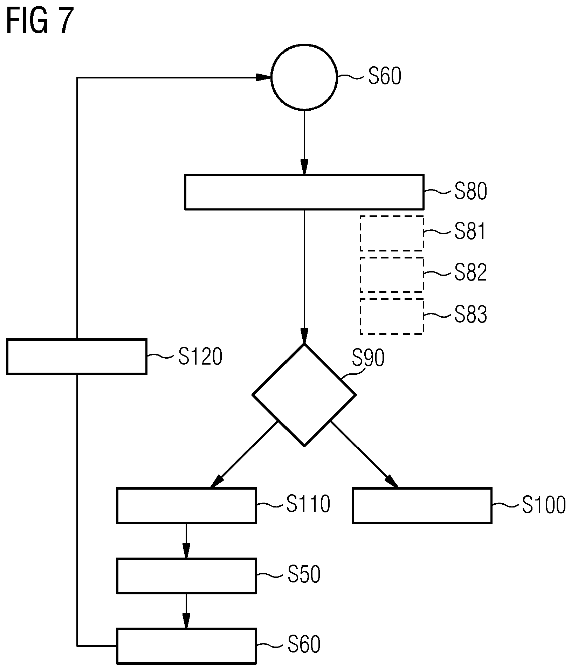

**Figure 7** depicts optional method steps for continuously checking a posture of patient P. The order of the steps does not necessarily correspond to the numbering of the steps but may also vary between different embodiments of the present invention. Further, individual steps or sequences of steps may be repeated.

The method steps of Figure 7 may for instance be implemented during the initial adjustment of patient support device 10 which may happen at step S60. Additionally, or alternatively, the method steps of Figure 7 may be used to constantly check the patient's P posture after patient support device 10 has already assumed the "final" spatial adjustment state as calculated in steps S30 and S40. In particular, the steps of Figure 7 may be applied during conducting the examination. With that, unwanted displacements of body segments of patient P, which may occur due to movements of patient P, may be recognized, and optionally compensated for.

At step S80 current positional information of at least one body segment of patient P is received at computing module 30. According to some examples, current positional information may be acquired using detection unit 50. If so, steps S80 may comprise optional sub step S81 directed to measure and provide detection results using detection unit 50. At subsequent optional step S82, the detection results are received at computing unit 30 via interface unit 20. At optional step S83, the detection results are processed to derive current positional information therefrom. Based on detection results from detection device 51, this may involve calculating one or more anatomical landmarks of patient P. These anatomical landmarks may preferably be calculated based on the three-dimensional contour mentioned earlier and in the coordinate system of patient support device 10 (in particular, patient table 12). Thereby, the position of the anatomical landmarks in the coordinate system of the patient support device 10 may be determined if a correlation between the coordinate system of the detection result recorded by detection device 51 and the coordinate system of patient support device 10 is known. Such a correlation can be ascertained by calibration which can precede the method according to the invention. In case of detection results from sensor device 52, pressure signals may be analyzed directly to infer how patient P is positioned on patient support device 10.

At step S90, the current positional information is compared to the posture determined at step S30. This may involve deriving a current posture for one or more body segments of patient P. Further, if the comparison reveals that there is no or a tolerable deviation between the current and the determined posture, computing unit 30 may ascertain that the determined posture has been reached. In that case computing unit 30 may provide a signal indicative the determined posture has been reached to the medical examination apparatus and/or a user of the positioning apparatus in optional step S100.

If the comparison at step S90 reveals that there is a deviation between the current and the determined posture the processing may move on to step S110. At step S100, control signals are generated based on this comparison. As before, the control signals are generally suited to control patient support device 10 such that it is adjusted such that the one or more body segments of the patient P are arranged and/or fixed in the determined posture at step S30. In particular, the control signals are suited to bring one or more body segments of patient P from a current posture to the determined posture. In doing so, deviations from the determined posture can systematically and actively be balanced. If the comparison reveals that there is no or a tolerable deviation between the current and the determined posture the control signals may be hold signals to just hold the current adjustment state.

At step S50, the control signals generated at step S100 are provided to patient support device 10 and at step S60 patient support device 10 is moved essentially as explained before. In this regard, step S120 is a repeat step indicating that the current positional information may constantly be acquired during the movement of patient support device 10 and used to readjust patient support device if needed.

Advantages which are described with respect to one embodiment of the present invention are, wherever applicable, also advantageous to other embodiments of the present invention.

## Claims

1. Positioning apparatus (100) for adjusting a posture of a patient (P) for an imaging and/or interventional and/or a therapeutic examination of the patient (P), comprising:
- a patient support device (10), the patient support device (10) being adjustable such that one or more body segments of the patient (P) may be arranged and/or fixed in a predetermined posture;
- an interface unit (20), configured to provide examination information (EI) indicative of an examination to be performed at the patient (P); and
- a computing unit (30) configured to:
▪ receive (S10) the examination information (EI) via the interface unit (20);
▪ determine (S30), based on the examination information (EI), a posture of one or more body segments of the patient (P) for the examination;
▪ generate (S40) one or more control signals (CS), the one or more control signals (CS) being suited to control the patient support device (10) such that the patient support device (10) is adjusted such that the one or more body segments of the patient (P) are arranged and/or fixed in the determined posture; and
provide (S50) the control signals (CS) to the patient support device (10), wherein:
- the examination information (EI) includes an information about a previous posture previously determined for the patient in a prior examination, wherein the information about the previous posture comprises medical image data depicting a region of interest targeted in the prior examination; and
- the computing unit (30) is configured to determine (S30) the posture by identifying a region of interest of the patient targeted in the prior examination from the information about the previous posture by processing the medical image data acquired in the prior examination and determining the posture based on the identified region of interest.

2. Positioning apparatus (100) according to claim 1, wherein:
the interface unit (20) is configured to be in data communication with a data base (41), in which data base (41) the examination information (EI) is stored; and
the interface unit (20) provides (S11) the examination information (EI) to the computing unit (30).

3. Positioning apparatus (100) according to any one of the preceding claims, wherein:
the interface unit (20) is further configured to provide (S21) patient information (PI), the patient information (PI) comprising one or more properties of the patient (P); and
the computing unit (30) is further configured to:
receive (S20) the patient information (PI) via the interface unit (20); and
determine (S30) the posture of one or more body segments of the patient(P) for the examination additionally based on the patient information (PI).

4. Positioning apparatus (100) according to claim 3, further comprising
a detection unit (50) configured to provide (S200) a detection result by measuring the patient (P) prior and/or during the examination and to provide the detection result to the interface unit (20); wherein:
the interface unit (20) is configured to provide (S200) the detection result to the computing unit (30); and
the computing unit (30) is configured to receive (S210) the detection result and to extract (S220) at least part of the patient information (PI) from the detection result.

5. Positioning apparatus (100) according to any one of the preceding claims, further comprising
a detection unit (50) configured to provide (S81) a detection result indicative of a current posture of one or more body segments of the patient (P) arranged on the patient support device (10) and to provide the detection result to the interface unit (20); wherein:
the interface unit (20) is configured to provide (S81) the detection result to the computing unit (30); and
the computing unit (30) is configured to receive (S82) the detection result, to extract (S83) an information about the current posture of one or more body segments of the patient (P) from the detection result, and to determine (S30) the one or more control signals additionally based on the information about the current posture.

6. Positioning apparatus (100) according to claims 4 or 5, wherein
the detection unit (50) includes a camera facility (51), a laser-scanning facility (51), and/or a pressure-sensitive sensor facility (52).

7. Positioning apparatus (100) according to any one of the preceding claims; wherein:
the computing unit (30) is configured to determine (S30) the posture such that the determined posture matches the previous posture.

8. Positioning apparatus (100) according to any one of the preceding claims; wherein:
the examination is conducted with a medical examination apparatus (60), the medical examination apparatus (60) comprising a predetermined operation range, wherein the medical examination apparatus (60) comprises a radiotherapy device (62) and the operation range is determined by the irradiation region of the radiotherapy device (62); and
the computing unit (30) is configured to determine (S30) the posture additionally based on the operation range.

9. Positioning apparatus (100) according to any one of the preceding claims, wherein
the computing unit (30) is configured to ascertain (S90) if the determined posture of the one or more body segments has been reached and, if so, providing (S100) a signal indicative the determined posture of the one or more body parts has been reached to the medical examination apparatus the examination is conducted with and/or a user of the positioning apparatus.

10. Positioning apparatus according to any one of the preceding claims, wherein
the computing unit (30) is further configured to:
host at least one machine-learned function configured to determine a posture of one or more body segments of a patient based on input data; and
determine (S30) the posture by applying the at least one machine-learned function to at least the examination information (EI).

11. Positioning apparatus (100) according to any one of the preceding claims; wherein:
the patient support device (10) comprises
a plurality of support segments (13) each configured to support different body segments of the patient (P), the support segments (13) being adjustable relative to one another such that one or more body segments of the patient (P) can be arranged and/or fixed at a predetermined posture; and
the one or more control signals are suited to adjust at least a part of the support segments (13) such that the one or more body segments of the patient (P) supported by the support segments (13) are arranged and/or fixed at the determined posture.

12. Positioning apparatus according to claim 11, wherein
at least some of the support segments (13) are provided with actuators (14) which can be activated by the control signals in order to adjust the associated support segments (13).

13. Medical examination system (1) comprising:
a positioning apparatus (100) according to any one of claims 1 to 12, and
a medical examination apparatus (60) for conducting the imaging and/or interventional and/or therapeutic examination.

14. Computer-implemented method to determine a posture of a patient (P) for an imaging and/or interventional and/or therapeutic examination to be performed on the patient (P), wherein the patient is arranged at a patient support device (10) during the procedure, the patient support device (10) being adjustable such that one or more body segments of the patient (P) can be arranged and/or fixed at a predetermined posture, the method comprising the following steps:
providing (S10) an examination information (EI) indicative of the examination to be performed at the patient (P), wherein the examination information (EI) includes an information about a previous posture previously determined for the patient in a prior examination, wherein the information about the previous posture comprises medical image data depicting a region of interest targeted in the prior examination;
determining (S30) a posture of one or more body segments of the patient based on the examination information (EI) by identifying a region of interest of the patient targeted in the prior examination from the information about the previous posture by processing the medical image data acquired in the prior examination and determining the posture based on the identified region of interest;
generating (S40) one or more control signals, the one or more control signals being suited to control the patient support device (10) such that the patient support device (10) is adjusted such that the one or more body segments of the patient (P) are arranged and/or fixed in the determined posture; and
providing (S50) the control signals to the patient support device (10).

15. Computer program product comprising program elements which induce a computing unit (30) of an positioning apparatus (100) for adjusting a posture of a patient (P) or a medical examination system (1) to perform the steps according to the method of claim 14, when the program elements are loaded into the computing unit (30).

## Patentansprüche

1. Positioniervorrichtung (100) zur Einstellung einer Körperhaltung eines Patienten (P) für eine bildgebende und/oder interventionelle und/oder eine therapeutische Untersuchung des Patienten (P), umfassend:
- eine Patiententragvorrichtung (10), wobei die Patiententragvorrichtung (10) derart einstellbar ist, dass ein oder mehrere Körpersegmente des Patienten (P) in einer vorbestimmten Körperhaltung angeordnet und/oder fixiert werden können;
- eine Schnittstelleneinheit (20), die dazu ausgelegt ist, Untersuchungsinformationen (EI) bereitzustellen, die eine an dem Patienten (P) durchzuführende Untersuchung angeben; und
- eine Recheneinheit (30), die dazu ausgelegt ist:
▪ die Untersuchungsinformationen (EI) über die Schnittstelleneinheit (20) zu empfangen (S10);
▪ basierend auf den Untersuchungsinformationen (EI) eine Körperhaltung von einem oder mehreren Körpersegmenten des Patienten (P) für die Untersuchung zu bestimmen (S30);
▪ ein oder mehrere Steuersignale (CS) zu erzeugen (S40), wobei das eine oder die mehreren Steuersignale (CS) dazu geeignet sind, die Patiententragvorrichtung (10) derart zu steuern, dass die Patiententragvorrichtung (10) derart eingestellt wird, dass das eine oder die mehreren Körpersegmente des Patienten (P) in der bestimmten Körperhaltung angeordnet und/oder fixiert werden; und
die Steuersignale (CS) der Patiententragvorrichtung (10) bereitzustellen (S50), wobei:
- die Untersuchungsinformationen (EI) Informationen über eine vorherige Körperhaltung aufweisen, die zuvor in einer vorherigen Untersuchung für den Patienten bestimmt wurde, wobei die Informationen über die vorherige Körperhaltung medizinische Bilddaten umfasst, die eine Interessensregion darstellen, auf die die vorherige Untersuchung ausgerichtet war; und
- die Recheneinheit (30) dazu ausgelegt ist, die Körperhaltung zu bestimmen (S30) durch Identifizieren einer Interessensregion des Patienten, auf die die vorherige Untersuchung ausgerichtet war, aus den Informationen über die vorherige Körperhaltung durch Verarbeiten der in der vorherigen Untersuchung gewonnenen Daten und Bestimmen der Körperhaltung basierend auf der identifizierten Interessensregion.

2. Positioniervorrichtung (100) nach Anspruch 1, wobei:
die Schnittstelleneinheit (20) dazu ausgelegt ist, mit einer Datenbank (41) in Datenverbindung zu stehen, in welcher Datenbank (41) die Untersuchungsinformationen (EI) gespeichert sind; und
die Schnittstelleneinheit (20) der Recheneinheit (30) die Untersuchungsinformationen (EI) bereitstellt (S11).

3. Positioniervorrichtung (100) nach einem der vorhergehenden Ansprüche, wobei:
die Schnittstelleneinheit (20) ferner dazu ausgelegt ist, Patienteninformationen (PI) bereitzustellen (S21), wobei die Patienteninformationen (PI) eine oder mehrere Eigenschaften des Patienten (P) umfassen; und
die Recheneinheit (30) ferner dazu ausgelegt ist:
die Patienteninformationen (PI) über die Schnittstelleneinheit (20) zu empfangen (S20); und
die Körperhaltung von einem oder mehreren Körpersegmenten des Patienten (P) für die Untersuchung zusätzlich basierend auf den Patienteninformationen (PI) zu bestimmen (S30).

4. Positioniervorrichtung (100) nach Anspruch 3, ferner umfassend
eine Erfassungseinheit (50), die dazu ausgelegt ist, ein Erfassungsergebnis bereitzustellen (S200) durch Messen des Patienten (P) vor und/oder während der Untersuchung und dazu, das Erfassungsergebnis der Schnittstelleneinheit (20) bereitzustellen, wobei:
die Schnittstelleneinheit (20) dazu ausgelegt ist, der Recheneinheit (30) das Erfassungsergebnis bereitzustellen (S200); und
die Recheneinheit (30) dazu ausgelegt ist, das Erfassungsergebnis zu empfangen (S210) und mindestens einen Teil der Patienteninformationen (PI) aus dem Erfassungsergebnis zu extrahieren (S220).

5. Positioniervorrichtung (100) nach einem der vorhergehenden Ansprüche, ferner umfassend:
eine Erfassungseinheit (50), die dazu ausgelegt ist, ein Erfassungsergebnis, das eine aktuelle Körperhaltung von einem oder mehreren Körpersegmenten des auf der Patiententragvorrichtung (10) angeordneten Patienten (P) angibt, bereitzustellen (S81) und dazu, der Schnittstelleneinheit (20) das Erfassungsergebnis bereitzustellen, wobei:
die Schnittstelleneinheit (20) dazu ausgelegt ist, der Recheneinheit (30) das Erfassungsergebnis bereitzustellen (S81); und
die Recheneinheit (30) dazu ausgelegt ist, das Erfassungsergebnis zu empfangen (S82), um Informationen über die aktuelle Körperhaltung von einem oder mehreren Körpersegmenten des Patienten (P) aus dem Erfassungsergebnis zu extrahieren (S83), und um das eine oder die mehreren Steuersignale zusätzlich basierend auf den Informationen über die aktuelle Körperhaltung zu bestimmen (S30).

6. Positioniervorrichtung (100) nach Anspruch 4 oder 5, wobei die Erfassungseinheit (50) eine Kameraeinrichtung (51), eine Laserscaneinrichtung (51) und/oder eine druckempfindliche Sensoreinrichtung (52) aufweist.

7. Positioniervorrichtung (100) nach einem der vorhergehenden Ansprüche, wobei:
die Recheneinheit (30) dazu ausgelegt ist, die Körperhaltung derart zu bestimmen (S30), dass die bestimmte Körperhaltung der vorherigen Körperhaltung entspricht.

8. Positioniervorrichtung (100) nach einem der vorhergehenden Ansprüche, wobei:
die Untersuchung mit einer medizinischen Untersuchungsvorrichtung (60) durchgeführt wird, wobei die medizinische Untersuchungsvorrichtung (60) einen vorbestimmten Betriebsbereich umfasst, wobei die medizinische Untersuchungsvorrichtung (60) eine Strahlentherapieeinrichtung (62) umfasst und der Betriebsbereich durch die Bestrahlungszone der Strahlentherapieeinrichtung (62) bestimmt wird; und
die Recheneinheit (30) dazu ausgelegt ist, die Körperhaltung zusätzlich basierend auf dem Betriebsbereich zu bestimmen (S30).

9. Positioniervorrichtung (100) nach einem der vorhergehenden Ansprüche, wobei
die Recheneinheit (30) dazu ausgelegt ist, zu ermitteln (S90), ob die bestimmte Körperhaltung des einen oder der mehreren Körpersegmente erreicht ist und, wenn ja, ein Signal bereitzustellen (S100), das der medizinischen Untersuchungsvorrichtung, mit der die Untersuchung durchgeführt wird, und/oder einem Anwender der Positioniervorrichtung angibt, dass die bestimmte Körperhaltung des einen oder der mehreren Körperteile erreicht ist.

10. Positioniervorrichtung nach einem der vorhergehenden Ansprüche, wobei
die Recheneinheit (30) ferner dazu ausgelegt ist:
mindestens eine maschinell erlernte Funktion zu beherbergen, die dazu ausgelegt ist, eine Körperhaltung von einem oder mehreren Körpersegmenten eines Patienten basierend auf Eingabedaten zu bestimmen; und
die Körperhaltung durch Anwenden der mindestens einen maschinell erlernten Funktion auf mindestens die Untersuchungsinformationen (EI) zu bestimmen (S30).

11. Positioniervorrichtung (100) nach einem der vorhergehenden Ansprüche, wobei:
die Patiententragvorrichtung (10) umfasst
eine Vielzahl von Tragsegmenten (13), die jeweils dazu ausgelegt sind, verschiedene Körpersegmente des Patienten (P) zu tragen, wobei die Tragsegmente (13) bezogen aufeinander derart einstellbar sind, dass ein oder mehrere Körpersegmente des Patienten (P) in einer vorbestimmten Körperhaltung angeordnet und/oder fixiert werden können; und
das eine oder die mehreren Steuersignale dazu geeignet sind, mindestens einen Teil der Tragsegmente (13) derart einzustellen, dass das eine oder die mehreren Körpersegmente des Patienten (P), die von den Tragsegmenten (13) getragen werden, in der bestimmten Körperhaltung angeordnet und/oder fixiert werden.

12. Positioniervorrichtung nach Anspruch 11, wobei mindestens einige der Tragsegmente (13) mit Betätigungseinrichtungen (14) bereitgestellt sind, die durch die Steuersignale betätigt werden können, um die damit verbundenen Tragsegmente (13) einzustellen.

13. Medizinisches Untersuchungssystem (1), umfassend:
eine Positioniervorrichtung (100) nach einem der Ansprüche 1 bis 12, und
eine medizinische Untersuchungsvorrichtung (60) zum Durchführen der bildgebenden und/oder interventionellen und/oder therapeutischen Untersuchung.

14. Computerimplementiertes Verfahren zum Bestimmen einer Körperhaltung eines Patienten (P) für eine bildgebende und/oder interventionelle und/oder therapeutische Untersuchung, die an dem Patienten (P) durchzuführen ist, wobei der Patient während des Vorgangs auf einer Patiententragvorrichtung (10) angeordnet ist, wobei die Patiententragvorrichtung (10) derart einstellbar ist, dass ein oder mehrere Körpersegmente des Patienten (P) in einer vorbestimmten Körperhaltung angeordnet und/oder fixiert werden können, wobei das Verfahren die folgenden Schritte umfasst:
Bereitstellen (S10) von Untersuchungsinformationen (EI), die die an dem Patienten (P) durchzuführende Untersuchung angeben, wobei die Untersuchungsinformationen (EI) Informationen über eine vorherige Körperhaltung aufweisen, die zuvor in einer vorherigen Untersuchung für den Patienten bestimmt wurde, wobei die Informationen über die vorherige Körperhaltung medizinische Bilddaten umfasst, die eine Interessensregion darstellen, auf die die vorherige Untersuchung ausgerichtet war;
Bestimmen (S30) einer Körperhaltung von einem oder mehreren Körpersegmenten des Patienten basierend auf Untersuchungsinformationen (EI) durch Identifizieren einer Interessensregion des Patienten, auf die die vorherige Untersuchung ausgerichtet war, aus den Informationen über die vorherige Körperhaltung durch Verarbeiten der medizinischen Bilddaten, die in der vorherigen Untersuchung erfasst wurden, und Bestimmen der Körperhaltung auf Basis der identifizierten Interessensregion;
Erzeugen (S40) von einem oder mehreren Steuersignalen (CS), wobei das eine oder die mehreren Steuersignale dazu geeignet sind, die Patiententragvorrichtung (10) derart zu steuern, dass die Patiententragvorrichtung (10) derart eingestellt wird, dass das eine oder die mehreren Körpersegmente des Patienten (P) in der bestimmten Körperhaltung angeordnet und/oder fixiert werden; und
Bereitstellen (S50) der Steuersignale für die Patiententragvorrichtung (10).

15. Computerprogrammprodukt, umfassend Programmelemente, die eine Recheneinheit (30) einer Positioniervorrichtung (100) zum Einstellen einer Körperhaltung eines Patienten (P) oder eines medizinischen Untersuchungssystems (1) dazu veranlassen, die Schritte gemäß dem Verfahren von Anspruch 14 durchzuführen, wenn die Programmelemente in die Recheneinheit (30) geladen werden.

## Revendications

1. Installation (100) de mise en position pour régler une posture d'un patient (P) pour une imagerie et/ou une intervention et/ou un examen thérapeutique du patient (P), comprenant :
- un dispositif (10) de support du patient, le dispositif (10) de support du patient étant réglable de manière à ce que un ou plusieurs segments du corps du patient (P) puissent être disposés et/ou fixés dans une posture déterminée à l'avance ;
- une unité (20) d'interface configurée pour donner une information (EI) d'examen indiquant un examen à effectuer sur le patient (P) ; et
- une unité (30) informatique configurée pour :
▪ recevoir (S10) l'information (EI) d'examen en passant par l'unité (20) d'interface ;
▪ déterminer (S30), sur la base de l'information (EI) d'examen, une posture d'un ou de plusieurs segments du corps du patient (P) pour l'examen ;
▪ créer (S40) un ou plusieurs signaux (CS) de commande, le un ou les plusieurs signaux (CS) de commande étant propres à commander le dispositif (10) de support du patient, de manière à régler le dispositif (10) de support du patient de façon à ce que l'un ou les plusieurs segments du corps du patient (P) soient disposés et/ou fixés dans la posture déterminée ; et
donner (S50) les signaux (CS) de commande au dispositif (10) de support du patient, dans laquelle :
- l'information (EI) d'examen comprend une information sur une posture précédente déterminée précédemment pour le patient dans un examen antérieur, dans laquelle l'information sur la posture précédente comprend une donnée d'image médicale dépeignant une région à laquelle on s'intéresse ciblée dans l'examen antérieur ; et
- l'unité (30) informatique est configurée pour déterminer (S30), la posture en identifiant une région à laquelle on s'intéresse du patient ciblée dans l'examen antérieur à partir de l'information sur la posture précédente en traitant la donnée d'image médicale acquise dans l'examen antérieur et en déterminant la posture sur la base de la région identifiée à laquelle on s'intéresse.

2. Installation (100) de mise en position suivant la revendication 1, dans laquelle :
l'unité (20) d'interface est configurée pour être en communication de données avec une base (41) de données, base (41) de données dans laquelle l'information (EI) d'examen est mise en mémoire ; et
l'unité (20) d'interface donne (S11) l'information (EI) d'examen à l'unité (30) informatique.

3. Installation (100) de mise en position suivant l'une quelconque des revendications précédentes, dans laquelle :
l'unité (20) d'interface est configurée en outre pour donner (S21) de l'information (PI) sur le patient, l'information (PI) sur le patient comprenant une ou plusieurs propriétés du patient (P) ; et
l'unité (30) informatique est configurée en outre pour :
recevoir (S20) l'information (PI) sur le patient en passant par l'unité (20) d'interface ; et
déterminer (S30) la posture d'un ou de plusieurs segments du corps du patient (P) pour l'examen sur la base supplémentairement de l'information (PI) sur le patient.

4. Installation (100) de mise en position suivant la revendication 3, comprenant en outre
une unité (50) de détection configurée pour donner (S200) un résultat de détection en mesurant le patient (P) avant et/ou pendant l'examen et pour donner le résultat de la détection à l'unité (20) d'interface ; dans laquelle :
l'unité (20) d'interface est configurée pour donner (S200) le résultat de la détection à l'unité (30) informatique ; et
l'unité (30) informatique est configurée pour recevoir (S210) le résultat de la détection et pour extraire (S220) au moins une partie de l'information (PI) sur le patient du résultat de la détection.

5. Installation (100) de mise en position suivant l'une quelconque des revendications précédentes, comprenant en outre
une unité (50) de détection configurée pour donner (S81) un résultat de la détection indicateur d'une posture en cours d'un ou de plusieurs segments du corps du patient (P) disposés sur le dispositif (10) de support du patient et pour donner le résultat de la détection à l'unité (20) d'interface ; dans laquelle :
l'unité (20) d'interface est configurée pour donner (S81) le résultat de la détection à l'unité (30) informatique ; et
l'unité (30) informatique est configurée pour recevoir (S82) le résultat de la détection, pour extraire (S83), du résultat de la détection, une information sur la posture en cours d'un ou de plusieurs segments du corps du patient (P) et pour déterminer (S30) le un ou les plusieurs signaux de commande supplémentairement sur la base de l'information sur la posture en cours.

6. Installation (100) de mise en position suivant les revendications 4 ou 5, dans laquelle
l'unité (50) de détection comprend un équipement (51) de caméra, un équipement (51) de balayage laser et/ou un équipement (52) de capteur sensible à la pression.

7. Installation (100) de mise en position suivant l'une quelconque des revendications précédentes ; dans laquelle :
l'unité (30) informatique est configurée pour déterminer (S30) la posture de manière à ce que la posture déterminée corresponde à la posture précédente.

8. Installation (100) de mise en position suivant l'une quelconque des revendications précédentes ; dans laquelle :
l'examen est conduit avec une installation (60) d'examen médical, l'installation (60) d'examen médical comprenant une gamme d'opération déterminée à l'avance, dans laquelle l'installation (60) d'examen médical comprend un dispositif (62) de radiothérapie et la gamme d'opération est déterminée par la région de rayonnement du dispositif (62) de radiothérapie ; et
l'unité (30) informatique est configurée pour déterminer (S30) la posture supplémentairement sur la base de la gamme d'opération.

9. Installation (100) de mise en position suivant l'une quelconque des revendications précédentes, dans laquelle
l'unité (30) informatique est configurée pour s'assurer (S90) que la posture déterminée du ou des plusieurs segments du corps a été atteinte et, s'il en est ainsi, pour donner (S100) un signal indiquant que la posture déterminée de la une ou des plusieurs parties du corps a été atteinte à l'installation d'examen médical, l'examen étant effectué par et/ou un utilisateur de l'installation de mise en position.

10. Installation de mise en position suivant l'une quelconque des revendications précédentes, dans laquelle
l'unité (30) informatique est configurée en outre pour :
loger au moins une fonction d'apprentissage automatique configurée pour déterminer une posture d'un ou de plusieurs segments du corps d'un patient sur la base de données d'entrée ; et
déterminer (S30) la posture en appliquant la au moins une fonction d'apprentissage automatique à au moins l'information (EI) d'examen.

11. Installation (100) de mise en position suivant l'une quelconque des revendications précédentes ; dans laquelle
le dispositif (10) de support du patient comprend
une pluralité de segments (13) de support configurés chacun pour supporter des segments différents du corps du patient (P), les segments (13) de support étant réglables les uns par rapport aux autres de manière à pouvoir disposer et/ou fixer en une posture déterminée à l'avance un ou plusieurs segments du corps du patient (P) ; et
les uns ou plusieurs signaux de commande sont propres à régler au moins une partie des segments (13) du support, de manière à disposer et/ou fixer en la posture déterminée le un ou les plusieurs segments du corps du patient (P) supportés par les segments (13) du support.

12. Installation de mise en position suivant la revendication 11, dans laquelle
au moins certains des segments (13) de support sont pourvus d'actionneurs (14), qui peuvent être activés par les signaux de commande afin de régler les segments (13) de support associés.

13. Système (1) d'examen médical comprenant :
une installation (100) de mise en position suivant l'une quelconque des revendications 1 à 12, et
une installation (60) d'examen médical pour conduire l'imagerie et/ou l'intervention et/ou l'examen thérapeutique.

14. Procédé mis en œuvre par ordinateur pour déterminer une posture d'un patient (P) pour une imagerie et/ou une intervention et/ou un examen thérapeutique à effectuer sur le patient (P),
dans lequel le patient est disposé sur un dispositif (10) de support du patient pendant la procédure, le dispositif (10) de support du patient étant réglable de manière à ce qu'un ou plusieurs segments du corps du patient (P) puissent être disposés et/ou fixés en une posture déterminée à l'avance, le procédé comprenant les stades suivants :
donner (S10) une information (EI) d'examen indiquant l'examen à effectuer sur le patient (P), dans lequel l'information (EI) d'examen comprend une information sur une posture précédente déterminée précédemment pour le patient dans un examen antérieur, dans lequel l'information sur la posture précédente comprend une donnée d'image médicale dépeignant une région à laquelle on s'intéresse ciblée dans l'examen antérieur ;
déterminer (S30) une posture d'un ou plusieurs segments du corps du patient sur la base de l'information (EI) d'examen en identifiant une région à laquelle on s'intéresse du patient ciblée dans l'examen antérieur à partir de l'information sur la posture précédente en traitant la donnée d'image médicale acquise dans l'examen antérieur et en déterminant la posture sur la base de la région identifiée à laquelle on s'intéresse ;
créer (S40) un ou plusieurs signaux de commande, le un ou les plusieurs signaux de commande étant propres à commander le dispositif (10) de support du patient, de manière à régler le dispositif (10) de support du patient de façon à ce que le un ou les plusieurs segments du corps du patient (P) soient disposés et/ou fixés dans la posture déterminée ; et
donner (S50) les signaux de commande au dispositif (10) de support du patient.

15. Produit de programme d'ordinateur comprenant des éléments de programme, qui induisent une unité (30) informatique d'une installation (100) de mise en position à régler la posture d'un patient (P) ou un système (1) d'examen médical pour effectuer les stades suivants le procédé de la revendication 14, lorsque les éléments de programme sont chargés dans l'unité (30) informatique.
